(19) Europäisches Patentamt
European Patent Office
Office européen des brevets

(11) Veröffentlichungsnummer: **0 288 976 B1**

(12) # EUROPÄISCHE PATENTSCHRIFT

(45) Veröffentlichungstag der Patentschrift: **01.06.94**

(51) Int. Cl.$^5$: **C07D 213/86**, C07D 405/14, C07D 409/14, C07D 417/14, C07D 471/04

(21) Anmeldenummer: **88106673.2**

(22) Anmeldetag: **26.04.88**

Die Akte enthält technische Angaben, die nach dem Eingang der Anmeldung eingereicht wurden und die nicht in dieser Patentschrift enthalten sind.

(54) **Mittel zum Schutz gegen Pflanzenkrankheiten.**

(30) Priorität: **29.04.87 CH 1629/87**

(43) Veröffentlichungstag der Anmeldung:
**02.11.88 Patentblatt 88/44**

(45) Bekanntmachung des Hinweises auf die Patenterteilung:
**01.06.94 Patentblatt 94/22**

(84) Benannte Vertragsstaaten:
**AT BE CH DE ES FR GB GR IT LI LU NL**

(56) Entgegenhaltungen:
**EP-A- 0 253 468**
**GB-A- 711 756**
**US-A- 3 962 260**

**CHEMICAL ABSTRACTS, Band 86, 1977, Seite 507, Zusammenfassung Nr. 106533s, Columbus, Ohio, US; N. PLE et al.: "Syntheses of pyrido-as-triazines"**

(73) Patentinhaber: **CIBA-GEIGY AG**
**Klybeckstrasse 141**
**CH-4002 Basel(CH)**

(72) Erfinder: **Kunz, Walter, Dr.**
**Buchenstrasse 9**
**CH-4104 Oberwil(CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

EP 0 288 976 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Isonicotinoylpyridinyl-hydrazin-Derivate der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

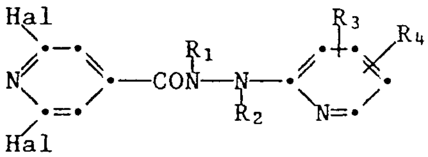

(I)

in welcher bedeuten:

Hal Halogen;

$R_1$ Wasserstoff, Methyl oder den Rest $COR_5$;

$R_2$ Wasserstoff oder einen der Reste $COR_5$ oder CO-Z-$R_6$;

$R_3$ Wasserstoff, Halogen, Trifluormethyl, Trichlormethyl, COOH, $COOCH_3$, OH oder Nitro;

$R_4$ Wasserstoff, Halogen, Methoxy oder Methyl;

$R_5$ $C_1$-$C_6$-Alkyl, ein- oder mehrfach mit Halogen substituiertes $C_1$-$C_6$-Alkyl, durch Sauerstoff oder Schwefel unterbrochenes $C_1$-$C_6$-Alkyl, ein- oder mehrfach mit Halogen substituiertes und durch Sauerstoff oder Schwefel unterbrochenes $C_1$-$C_6$-Alkyl, $C_2$-$C_4$-Alkenyl, ein- oder mehrfach mit Halogen substituiertes $C_2$-$C_4$-Alkenyl, Phenyl, durch Halogen, Methyl, Trifluormethyl oder Trichlormethyl substituiertes Phenyl, Benzyl, durch Halogen, Methyl, Trifluormethyl oder Trichlormethyl substituiertes Benzyl, ein 5- oder 6-gliedriger Heterocyclus mit Stickstoff, Sauerstoff oder Schwefel als Heteroatome, ein 3- bis 6-gliedriges Cycloalkyl oder ein einfach- oder mehrfach durch Halogen oder Methyl substituiertes Cycloalkyl;

$R_6$ $C_1$-$C_5$-Alkyl, Phenyl oder falls Z die CO-Gruppe darstellt $OC_1$-$C_2$-Alkyl; und

Z Sauerstoff, Schwefel oder die CO-Gruppe; mit Ausnahme von 1-(Pyridin-2'yl)-2-(2,6-dichlorisonicotinoyl)-hydrazin.

Halogen selbst oder als Bestandteil eines anderen Substituenten bedeutet Fluor, Chlor, Brom oder Jod, vorzugsweise Fluor, Chlor oder Brom.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3).

Cycloalkyl bedeutet wahlweise Cyclopropyl, Cyclobutyl, Cyclopentyl oder Cyclohexyl, vorzugsweise Cyclopropyl, Cyclopentyl oder Cyclohexyl. Unter den substituierten Cycloalkylresten ist 2,2-Dimethyl-3,3-dichlorcyclopropyl bevorzugt.

Unter den 5- oder 6-gliedrigen Heterocyclen mit Stickstoff, Sauerstoff und/oder Schwefel als Heteroatome sind z.B. bevorzugt: Thiophen, Thiazol, Furan, Pyridin, Thiadiazol, darunter 1,2,3-Thiadiazol.

Aufgrund ihrer ausgeprägten Pflanzen schützenden mikrobiziden Eigenschaften sind diejenigen Wirkstoffe der Formel I bevorzugt, die folgende Substituenten oder Kombinationen dieser Substituenten untereinander aufweisen:

1) Hal bedeutet Chlor oder Brom;

$R_1$ ist Wasserstoff;

$R_2$ stellt Wasserstoff oder eine der Gruppen $COCH_3$, $COC_2H_5$ oder

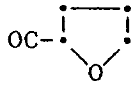

dar; und

$R_3$ und $R_4$ stehen unabhängig voneinander für Wasserstoff, Halogen oder Trifluormethyl.
2) Hal bedeutet Chlor;
$R_1$ ist Wasserstoff;
$R_2$ stellt Wasserstoff dar; und
$R_3$ und $R_4$ stehen unabhängig voneinander für Wasserstoff, Halogen oder Trifluormethyl.
3) Hal bedeutet Chlor;
$R_1$ ist Wasserstoff;
$R_2$ stellt eine der Gruppen $COCH_3$, $COC_2H_5$ oder

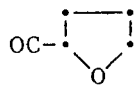

dar; und
$R_3$ und $R_4$ stehen unabhängig voneinander für Wasserstoff, Halogen oder Trifluormethyl.
4) Hal bedeutet Chlor;
$R_1$ ist Wasserstoff;
$R_2$ stellt Wasserstoff dar;
$R_3$ steht für Wasserstoff oder 3-Chlor; und $R_4$ steht für Wasserstoff oder 5-Trifluormethyl.
5) Hal bedeutet Chlor;
$R_1$ ist Wasserstoff;
$R_2$ stellt eine der Gruppen $COCH_3$, $COC_2H_5$ oder

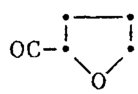

dar;
$R_3$ steht für Wasserstoff oder 3-Chlor; und
$R_4$ steht für Wasserstoff oder 5-Trifluormethyl.

Wegen ihrer hervorragenden biologischen Aktivität sind folgende Verbindungen bevorzugt:
1-Acetyl-1-pyridin-2'-yl)-2-(2,6-dichlorisonicotinoyl)-hydrazin;
1-Propionyl-1-pyridin-2'-yl)-2-(2,6-dichlorisonicotinoyl)-hydrazin;
1-Tetrahydrofuroyl-1-pyridin-2'-yl)-2-(2,6-dichlorisonicotinoyl)-hydrazin;
1-(3'-Chlor-5'-trifluormethylpyridin-2'-yl)-(2,6-dichlorisonicotinoyl)-hydrazin;
1-Methyl-1-(3'-chlor-5'-trifluormethylpyridin-2'-yl)-(2,6-dichlorisonicotinoyl)-hydrazin;
1-Ethyl-1-(3'-chlor-5'-trifluormethylpyridin-2'-yl)-(2,6-dichlorisonicotinoyl)-hydrazin.

Die Verbindung 1-(Pyridin-2'-yl)-2-(2,6-dichlorisonicotinoyl)-hydrazin ist bereits beschrieben. Ueber ihre biologischen Eigenschaften ist jedoch bisher nichts bekannt geworden. Sie hat sich nun überraschenderweise als sehr aktiv gegen Pflanzenkrankheiten erwiesen. Sie stellt als Wirkstoff in Form von Mitteln zum Schutz von Pflanzen gegen Krankheitsbefall sowie mit ihrer Verwendung zu diesem Zweck einen Bestandteil der vorliegenden Erfindung dar.

Die Verbindungen der Formel I werden erfindungsgemäss hergestellt, indem man entweder
a) 2-Hydrazino-pyridin-Derivate der Formel II

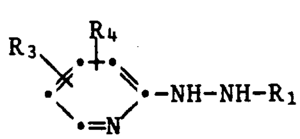

(II)

mit 2,6-Dihaloisonicotinoyl-Derivaten der Formel III

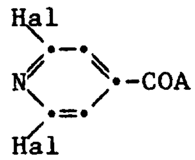

(III)

in einem inerten Lösungsmittel oder

b) 2,6-Dihaloisonicotinsäurehydrazid-Derivate der Formel IV

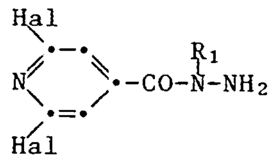

(IV)

mit substituierten 2-Halopyridin-Derivaten der Formel V

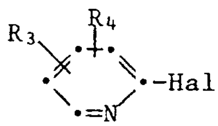

(V)

in einem inerten Lösungsmittel gegebenenfalls in Gegenwart eines Katalysators umsetzt, und anschliessend

c) die erhaltenen Verbindungen der Formel Ia

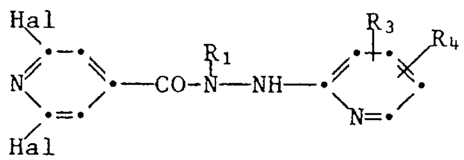

(Ia)

mit Verbindungen der Formel VI

$R_2CO$-Y (VI)

in einem inerten Lösungsmittel gegebenenfalls in Gegenwart einer Base reagieren lässt, wobei A Halogen, O-$C_1$-$C_4$-Alkyl oder S-$C_1$-$C_4$-Alkyl darstellt und Y Halogen oder O-$C_1$-$C_4$-Alkyl bedeutet und Hal und $R_1$ bis $R_4$ die in der Einleitung unter Formel I angegebenen Bedeutungen besitzen.

Die Reaktionstemperaturen der einzelnen Verfahrensstufen betragen für (a) und (c) 0° bis 180°C, vorzugsweise 20° bis 80°C, und für (b) -10° bis 180°C, vorzugsweise 0° bis 70°C.

In der Verfahrensstufe (c) ist die Verwendung von säurebindenden Mitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Alkoholate wie z.B. Kaliumtert.-Butylat, Oxide und Hydroxide, Carbonate und Hydrogencarbonate von Alkali- und Erdalkalimetallen sowie Alkaliacetate.

Für die Reaktion der Verfahrensstufe (b) kann sich die Verwendung eines Katalysators als günstig erweisen. Geeignete Katalysatoren sind beispielsweise Kupfersalze, vorzugsweise Kupfer(I)- oder Kupfer(II)-chlorid oder Kupfer(I)- oder Kupfer(II)acetat.

In den Verfahrensstufen (a) bis (c) werden als Reaktionsmedien in Anpassung an die jeweiligen Reaktionsbedingungen geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Herstellung der Ausgangsstoffe kann wie folgt durchgeführt werden:

Die 2-Hydrazino-pyridine der Formel II werden durch Umsetzung entsprechend substituierter 2-Halopyridine mit Hydrazin oder Methylhydrazin hergestellt. Dabei finden bevorzugt 2-Chlor- oder 2-Brom-pyridine Anwendung. Die Reaktion wird üblicherweise in einem Methanol/Wasser-Gemisch vorgenommen, wobei sie durch eine Katalyse mit ein- oder zweiwertigen Schwermetall-Ionen wie Cu(I)- oder Cu(II)-Salzen günstig beeinflusst werden kann (Lit.: Eur. J. Med. Chem. 10 (1975) 252; ferner J. Org. Chem. 31, 260).

Verbindungen der Formel III werden erhalten aus 2,6-Dihydroxyisonicotinsäure durch Behandlung mit Phosphoroxyhalogenid und gegebenenfalls durch Veresterung mit dem entsprechenden Alkohol. Die Umsetzung mit Phosphoroxyhalogenid findet bei Temperaturen von 50° - 200°C und einem Druck von 1-$100 \cdot 10^5$ Pa gegebenenfalls in Gegenwart einer Base statt.

Die Isonicotinsäurehydrazide der Formel IV werden z.B. herstellt durch Umsetzung von 2,6-Dihaloisonicotinsäurealkylestern mit Hydrazin oder Methylhydrazin bei Temperaturen von 0° - 100°C unter Normaldruck (sowie weitere Methoden vgl. US-PS 4,137,067; ferner Eur. J. Med. Chem. - Chimica Therap. 10 - (1975) 252).

Weitere Methoden zur Herstellung der Vorstufen-Produkte, darunter auch diejenigen der substituierten 2-Halopyridin-Derivate, sind dem Fachmann geläufig oder in der Fachliteratur beschrieben.

Pyridinylhydrazin-Derivate sind bereits als Wirkstoffe zur Anwendung auf verschiedenen Gebieten beschrieben worden. So z.B. als Fungizide und Bakterizide in der US-Patentschrift 3,962,260, ferner als Tuberculostatica in Acta Fac. Pharm. Brun. Bratislav. 4, 65-95 (1962) [Chem. Abstr. Vol. 57 (1962) 4769a] sowie als Mikrobizide in der US-Patentschrift 4,137,067.

Es wurde nun überraschend gefunden, dass die erfindungsgemässen Verbindungen der Formel I ein in der Praxis sehr günstiges Wirkungsspektrum zum Schutz von Pflanzen gegen Krankheiten entfalten, die sowohl von Fungi als auch von Bakterien und Viren hervorgerufen werden. Die den erfindungsgemässen Verbindungen zugrundeliegende Wirkungsweise ist vor allem auf eine allgemeine Erhöhung der Abwehrbereitschaft der behandelten Pflanzen gerichtet, wodurch eine generelle antimikrobielle Resistenz gegen ein breites Spektrum von schädlichen Mikroorganismen erzielt wird. Der grosse Vorteil der erfindungsgemässen Verbindungen besteht darin, dass bei ihrer Anwendung zur Behandlung von Pflanzen anstelle der direkten Einwirkung auf die pflanzenschädigenden Mikroorganismen eine Aktivierung und Stimulierung des pflanzeneigenen biologischen Abwehrsystems vor dem Krankheitsbefall der Pflanzen eintritt, so dass eine Gesunderhaltung der behandelten Pflanzen aus eigener Kraft ohne weiteren direkten Einsatz mikrobizider Substanzen während der Vegetationsperiode gewährleistet werden kann. Für die Wirkstoffe der Formel I ist somit charakteristisch, dass sie keine direkte Wirkung auf die Mikroorganismen ausüben, sondern stattdessen eine immunisierende Wirkung auf gesunde Pflanzen gegen Pflanzenkrankheiten besitzen. Die daraus resultierende Immunisierung gegen Pflanzen-Krankheiten lässt sich zum Schutz von zahlreichen Kulturpflanzen einsetzen, so dass an Pflanzen oder Pflanzenteilen (Früchte, Blüten, Laubwerk, Stengel, Knollen, Wurzeln) von unterschiedlichen Nutzkulturen das Auftreten der Schadorganismen wirksam verhindert wird, wobei auch später zuwachsende Pflanzenteile von phytopathogenen Mikroorganismen verschont bleiben. Im Gegensatz dazu ist jedoch bei einer Reihe von Verbindungen der Formel I auch deren protektiver Einsatz gegen phytopathogene Mikroorganismen möglich. Die Wirkstoffe der Formel I entfalten ihre Aktivität sowohl über die Blattapplikation als auch systemisch. Sie können ferner als Beizmittel zur Behandlung von Saatgut (Früchte, Knollen, Körner) und Pflanzenstecklingen zum Schutz gegen Pilzinfektionen, so z.B. gegen Fusarium nivale, Helminthosporum gramineum, Ustilago nuda, sowie gegen im Erdboden auftretende phytopathogene Mikroorganismen eingesetzt werden.

Das Wirkungsspektrum der Verbindungen der Formel I erstreckt sich z.B. auf phytopathogene Pilze folgender Klassen: Fungi imperfecti (z.B. Botrytis, Pyricularia, Colletotrichum, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula) und Phycomyceten (z.B. Phytophthora, Plasmopara). Darüber hinaus wirken die erfindungsgemässen Verbindungen gegen phytopathogene Bakterien und Viren (z.B. gegen Xanthomonas spp, Pseudomonas spp., Erwinia amylovora sowie gegen den Tabakmosaik-Virus).

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen

Substanzen bzw. Substanzgruppen. Eingeschlossen ist auch ein Verfahren zur Behandlung von Pflanzen, das sich durch Applikation der neuen Verbindungen der Formel I bzw. der diese enthaltenden neuen Mittel auszeichnet.

Als Zielkulturen für den hierin offenbarten pflanzenschützenden Einsatz gelten im Rahmen dieser Erfindung beispielsweise folgende Plfanzenarten: Getreide (Weizen, Gerste, Roggen, Hafer, Reis, Mais, Sorghum und Verwandte); Rüben (Zucker- und Futterrüben); Kern-, Stein- und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Hohn, Oliven, Sonnenblumen, Kokos, Rizinus, Kakao, Erdnüsse); Gurkengewächse (Kürbis, Gurken, Melonen); Fasergewächse: (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen sowie die Behandlung von Reispflanzen mittels der sogenannten "into water application".

Die Verbindungen der Formel I werden dabei in unveränderter Form oder vorzugsweise zusammen mit den in der Formulierungstechnik üblichen Hilfsmittel eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwaserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch Sepiolit oder Bentonit, als nicht sorptive Trägermaterialien, z.B. Calcit oder Sand, in

Frage. Darüber hinaus kann eine Vielzahl von vorgranulierten Materialien anorganischer oder organischer Natur wie insbesondere Dolomit oder zerkleinerte Pflanzenrückstände verwendet werden. Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Agrochemische Mittel, die Verbindungen der Formel I als Aktivkomponente enthalten, stellen ebenfalls einen Bestandteil der vorliegenden Erfindung dar.

Die nachfolgenden Beispiele dienen zur näheren Erläuterung der Erfindung, ohne dieselbe einzuschränken.

1. Herstellungsbeispiele

Beispiel 1.1: Herstellung von

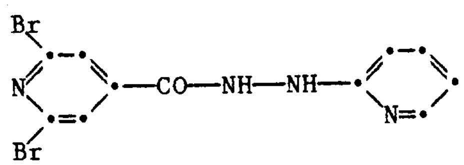

1-(Pyridin-2'-yl)-2-(2,6-dibromisonicotinoyl)-hydrazin

6,2 g 2-Hydrazino-pyridin werden in 50 ml absolutem Pyridin gelöst und unter Rühren tropfenweise mit einer Lösung von 17,6 g 2,6-Dibromisonicotinsäurechlorid in 10 ml absolutem Acetonitril versetzt. Durch Kühlen wird die Reaktionstemperatur unter 35 °C gehalten. Nach Abklingen der Reaktion wird aufgeheizt und während 7 Std. auf 65 °C gehalten. Dann wird abgekühlt, auf ca. 100 ml Eiswasser gegossen und der entstehende Niederschlag abfiltriert und mit Wasser und Hexan/Diethylether (8:2) nachgewaschen. Nach Trocknen unter Vakuum erhält man 17,4 g weisse Kristalle mit einem Smp. von 187-190 °C.

Beispiel 1.2: Herstellung von

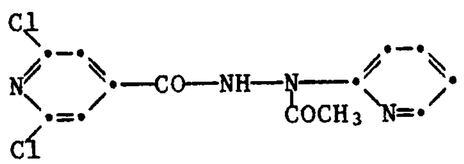

1-(Acetyl-1-pyridin-2'-yl)-2-(2,6-dichlorisonicotinoyl)-hydrazin

4,2 g der Verbindung von Beispiel 1.1 werden in Portionen in 20 ml Acetanhydrid eingetragen und anschliessend die erhaltene gelbe Suspension während 2 Std. auf 85 °C erwärmt, wobei Lösung eintritt. Ueberschüssiges Acetanhydrid wird unter Vakuum schnell abdestilliert und der Rückstand durch Zugabe von wenig Diethylether/Petrolether zur Kristallisation gebracht. Der Niederschlag wird abgesaugt und mit Petrolether gewaschen. Es resultieren 4,2 g der Titelverbindung mit einem Smp. von 128-130 °C.

Beispiel 1.3: Herstellung von

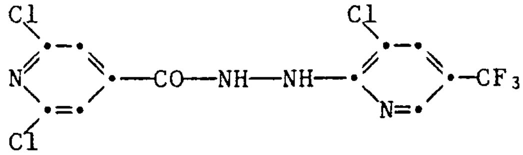

1-(3-Chlor-5-trifluoromethylpyridin-2'-yl)-2-(2,6-dichlorisonicotinoyl)-hydrazin

50,2 g 2,6-Dichlorisonicotinsäurehydrazid werden in 250 ml Tetrahydrofuran aufgeschlämmt und mit 14 g fein pulveriertem Kaliumhydroxid versetzt. Bei 0-5 °C werden 0,8 g Kupfer-II-acetat zugegeben und innerhalb einer 3/4 Std. eine Lösung von 57,5 g 2,3-Dichlor-5-trifluormethylpyridin in 150 ml Tetrahydrofuran zugetropft. Danach wird das Kühlbad entfernt und das Reaktionsgemisch über Nacht am Rückfluss

gekocht. Anschliessend werden nochmals 0,8 g Kupfer-II-acetat und 6 g 2,3-Dichlor-5-trifluormethylpyridin zugegeben und weitere 24 Std. am Rückfluss gekocht. Dann wird das Reaktionsgemisch unter Vakuum auf die Hälfte des Volumens eingedampft und auf Eiswasser gegossen. Nach Extraktion mit Dichlormethan und Waschen mit Wasser wird erneut eingedampft und über eine Kieselgelsäule (Essigsäureethylester-Diethylether 1:1) gereinigt. Man erhält die Titelverbindung in Form beiger Kristalle mit einem Smp. von 154-155 °C.

Beispiel 1.4: Herstellung von

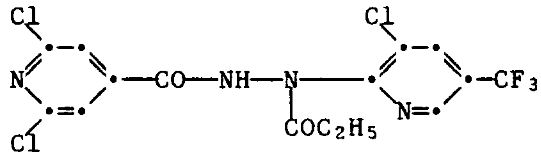

1-Propionyl-1-(3-chlor-5-trifluoromethylpyridin-2'-yl)-2-(2,6-dichlorisonicotinoyl)-hydrazin

5,8 g 2-(2,6-Dichlorisonicotinoyl)-1-(3-chlor-5-trifluormethylpyridin-2'-yl)-hydrazin in 45 ml Tetrahydrofuran werden 16,5 ml 1N Natronlauge gegeben und unter Rühren bei Raumtemperatur eine Lösung von 1,5 ml Propionsäurechlorid in 4,5 ml Tetrahydrofuran zugetropft. Ueber Nacht wird bei Raumtemperatur gerührt, anschliessend nochmals je 25 % der oben angegebenen Mengen an 1N Natronlauge und Propionsäurechlorid zugegeben. Nach total 48 Std. Reaktionszeit wird mit Wasser verdünnt, mit Essigsäureethylester extrahiert, die Extrakte mit Wasser gewaschen, getrocknet und eingedampft. Der kristalline Rückstand (6 g ≙ 90 % der Theorie) wird mit wenig Diethylether angeschlämmt und abfiltriert. Das reine Produkt hat einen Smp. von 168 °-170 °C.

Gemäss den beschriebenen Herstellungsweisen werden die nachfolgend aufgeführten Verbindungen erhalten:

Tabelle 1: Verbindungen der Formel

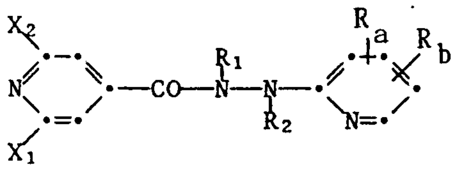

| Verb. Nr. | $X_1$ | $X_2$ | $R_1$ | $R_2$ | $R_a$ | $R_b$ | physik. Daten |
|---|---|---|---|---|---|---|---|
| 1.1 | Cl | Cl | H | $COCH_3$ | H | H | Fp. 128-130°C |
| 1.2 | Cl | Cl | $CH_3$ | $COCH_3$ | H | H | |
| 1.3 | Cl | Cl | H | $COCH_2CH_3$ | H | H | Fp. 137-139°C |
| 1.4 | Cl | Cl | H | $COCH_3$ | 3-Cl | 5-$CF_3$ | Fp. 177-179°C |
| 1.5 | Cl | Cl | H | $COCH_2OCH_3$ | H | H | Fp. 48-51°C |
| 1.6 | Cl | Cl | H | $COC(CH_3)_3$ | H | H | Fp. 182-185°C |
| 1.7 | Cl | Cl | H | $COCH_2$-$C_6H_5$ | H | H | Fp. 189-190°C |
| 1.8 | Cl | Cl | H | CO– 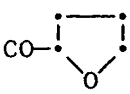 | H | H | Fp. 164-166°C |
| 1.9 | Cl | Cl | H | CO–$CH_2$– 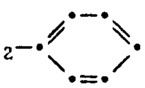 –Br | H | H | Fp. 170-172°C |
| 1.10 | Cl | Cl | H | CO– 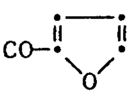 | H | H | Fp. 163-165°C |
| 1.11 | Br | Br | H | $COCH_3$ | H | H | Fp. 157-160°C |
| 1.12 | Cl | Cl | H | CO–$CH_2$– 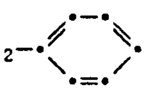 –$CH_3$ | 3-Cl | 5-$CF_3$ | Fp. 174-177°C |
| 1.13 | Cl | Cl | H | $COCH$=$CH$-$CH_3$ | 3-Cl | 5-$CF_3$ | Fp. 173-176°C |
| 1.14 | Cl | Cl | H | CO-$C_6H_{13}$-n | H | H | Fp. 105-107°C |
| 1.15 | Cl | Cl | H | $COCH_2CH_3$ | 3-Cl | 5-$CF_3$ | $n_D^{50}$ = 1.5330 |

Tabelle 1: (Fortsetzung)

| Verb. Nr. | $X_1$ | $X_2$ | $R_1$ | $R_2$ | $R_a$ | $R_b$ | physik. Daten |
|---|---|---|---|---|---|---|---|
| 1.16 | Cl | Cl | H | 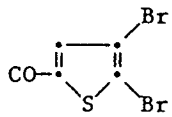 | H | H | Fp. 204-207°C |
| 1.17 | Cl | Cl | H | 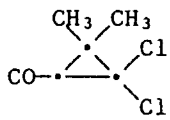 | H | H | |
| 1.18 | Br | Br | H | 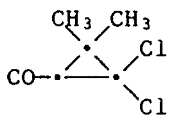 | 3-Cl | 5-$CF_3$ | |
| 1.19 | Cl | Cl | H | $COCH_3$ | 4-$CF_3$ | 6-Cl | |
| 1.20 | Cl | Cl | H | $COCH_3$ | 4-$CCl_3$ | 6-Cl | |
| 1.21 | Cl | Cl | H | $COCH_3$ | 4-$CCl_3$ | 6-$OCH_3$ | |
| 1.22 | Cl | Cl | H | $COC_2H_5$ | 4-$COOCH_3$ | 6-Cl | |
| 1.23 | Cl | Cl | H | $COCH_2OCH_3$ | 4-$COOCH_3$ | 6-Br | |
| 1.24 | J | J | H | 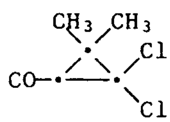 | H | H | |
| 1.25 | Cl | Cl | H | $COOCH_3$ | H | H | |
| 1.26 | F | F | H | $COOC_2H_5$ | 3-Cl | 5-$CF_3$ | |
| 1.27 | F | F | H | COO-$C_6H_5$ | H | H | |
| 1.28 | Br | Br | H | 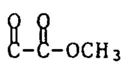 | H | H | |
| 1.29 | Cl | Cl | H | 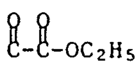 | 3-Cl | 5-$CF_3$ | |
| 1.30 | Cl | Cl | H | $COSCH_3$ | H | H | |
| 1.31 | Cl | Cl | $CH_3$ | $COOCH_3$ | H | H | |
| 1.32 | Cl | Cl | H | 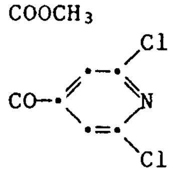 | H | H | 204-208°C |
| 1.33 | Cl | Cl | $CH_3$ | 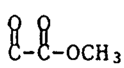 | H | H | |
| 1.34 | Cl | Cl | H | $COCH_3$ | 3-Cl | 5-Cl | |
| 1.35 | Br | Br | H | $COCH_3$ | 3-Cl | 5-Cl | |
| 1.36 | F | F | H | $COCH_3$ | 3-Cl | 5-Cl | |

Tabelle 1: (Fortsetzung)

| Verb. Nr. | $X_1$ | $X_2$ | $R_1$ | $R_2$ | $R_a$ | $R_b$ | physik. Daten |
|---|---|---|---|---|---|---|---|
| 1.37 | Cl | Cl | H | $COCH_3$ | 5-$CH_3$ | H | |
| 1.38 | Br | Br | H | $COC_2H_5$ | 5-$CH_3$ | H | |
| 1.39 | Cl | Cl | $CH_3$ | $COCH_3$ | 3-Cl | 5-$CH_3$ | |
| 1.40 | Cl | Cl | H | $COCH_3$ | 3-Cl | 5-$CH_3$ | |
| 1.41 | Cl | Cl | H | $COCH_3$ | 3-$CF_3$ | 6-Cl | |
| 1.42 | Cl | Cl | H | $COCH=CHCH_3$ | H | H | Fp. 112-115°C |
| 1.43 | Cl | Cl | $CH_3$ | $COCH_3$ | H | H | |
| 1.44 | Cl | Cl | $CH_3$ | $COCH_3$ | 3-Cl | 5-$CF_3$ | |
| 1.45 | Br | Br | $CH_3$ | 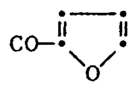 | H | H | |
| 1.46 | J | J | $CH_3$ | $COC_2H_5$ | H | H | |
| 1.47 | Cl | Cl | H | $COCH_2Cl$ | 3-Cl | 5-$CF_3$ | |
| 1.48 | Cl | Cl | H | $COCCl_3$ | H | H | |
| 1.49 | Cl | Cl | H | $COCHCl_2$ | H | H | |
| 1.50 | Cl | Cl | H | $COCH_2SCH_3$ | H | H | |
| 1.51 | Cl | Cl | H | $CO(CH_2)_2SCH_3$ | 3-Cl | 5-$CF_3$ | |
| 1.52 | Br | Br | H | 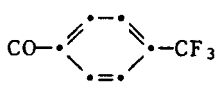 | H | H | |
| 1.53 | J | J | H | 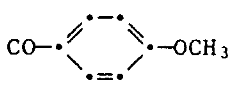 | 3-Cl | 5-$CF_3$ | |
| 1.54 | Cl | Cl | H | 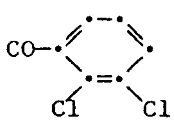 | H | H | |
| 1.55 | Cl | Cl | H | 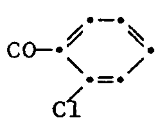 | 6-Cl | H | |
| 1.56 | J | J | H | 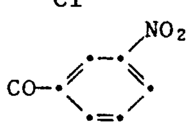 | H | H | |
| 1.57 | F | F | H | 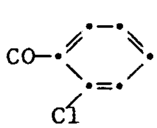 | H | H | |

Tabelle 1: (Fortsetzung)

| Verb. Nr. | $X_1$ | $X_2$ | $R_1$ | $R_2$ | $R_a$ | $R_b$ | physik. Daten |
|---|---|---|---|---|---|---|---|
| 1.58 | Cl | Cl | H | 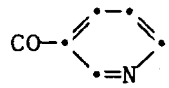 | H | H | |
| 1.59 | Cl | Cl | H | 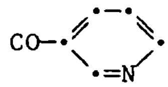 | 3-Cl | 5-$CF_3$ | |
| 1.60 | Cl | Cl | $CH_3$ | 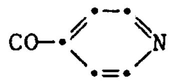 | 3-Cl | H | |
| 1.61 | Cl | Cl | H | 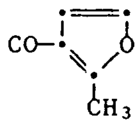 | H | H | |
| 1.62 | Cl | Cl | H | 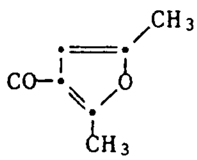 | H | H | |
| 1.63 | Cl | Cl | H | 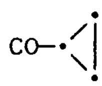 | 3-Cl | 5-$CF_3$ | |
| 1.64 | Cl | Cl | H | 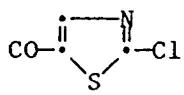 | H | H | |
| 1.65 | Cl | Cl | $CH_3$ | 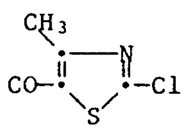 | H | H | |
| 1.66 | Cl | Cl | H | 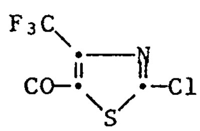 | H | H | |
| 1.67 | Cl | Cl | H | 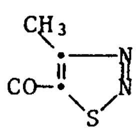 | H | H | |
| 1.68 | Cl | Cl | H | 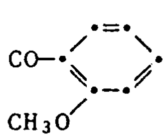 | H | H | |

Tabelle 1: (Fortsetzung)

| Verb. Nr. | $X_1$ | $X_2$ | $R_1$ | $R_2$ | $R_a$ | $R_b$ | physik. Daten |
|---|---|---|---|---|---|---|---|
| 1.69 | Cl | Cl | H | 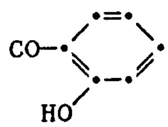 | H | H | |
| 1.70 | Cl | Cl | H | 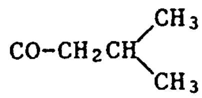 | H | H | Fp. 123-126°C |
| 1.71 | Cl | Cl | H | $CO-CH_2OCH_3$ | 3-Cl | $5-CF_3$ | Fp. 139-142°C |
| 1.72 | Cl | Cl | H | $CO(CH_2)_2CH_3$ | H | H | Fp. 148-150°C |
| 1.73 | Cl | Cl | * | $CO(CH_2)_5CH_3$ | H | H | Fp. 90-92°C |
| 1.74 | Cl | Cl | ** | $COCH_3$ | H | H | Fp. 116-117°C |
| 1.75 | Cl | Cl | H | $COCH_3$ | $5-NO_2$ | H | |
| 1.76 | Cl | Cl | H | $COC_2H_5$ | $5-NO_2$ | H | |
| 1.77 | Br | Br | H | $COC_2H_5$ | $5-NO_2$ | H | |

* $CO(CH_2)_5CH_3$

** $COCH_3$

Tabelle 2: Verbindungen der Formel

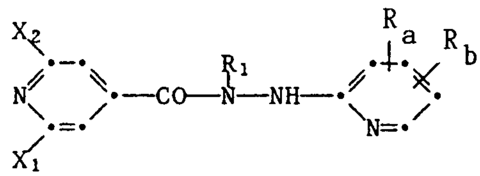

| Verb. Nr. | $X_1$ | $X_2$ | $R_1$ | $R_a$ | $R_b$ | physik. Daten |
|---|---|---|---|---|---|---|
| 2.1 | Cl | Cl | H | H | H | Fp. 196-199°C |
| 2.2 | Cl | Cl | H | 3-Cl | 5-$CF_3$ | Fp. 154-155°C |
| 2.3 | Br | Br | H | H | H | Fp. 187-190°C |
| 2.4 | Br | Br | H | 3-Cl | 5-$CF_3$ | |
| 2.5 | Cl | Cl | H | 3-Cl | 5-$CH_3$ | |
| 2.6 | Cl | Cl | H | 4-$CCl_3$ | 6-Cl | |
| 2.7 | Cl | Cl | H | 4-$CCl_3$ | 6-$OCH_3$ | |
| 2.8 | Cl | Cl | H | 4-$COOCH_3$ | 6-Cl | |
| 2.9 | J | J | H | H | H | |
| 2.10 | J | J | H | 3-Cl | 5-$CF_3$ | |
| 2.11 | F | F | H | H | H | |
| 2.12 | F | F | H | 3-Cl | 5-$CF_3$ | |
| 2.13 | Cl | Cl | H | 3-$CF_3$ | 6-Cl | |
| 2.14 | Cl | Cl | H | 5-$CH_3$ | H | |
| 2.15 | Br | Br | H | 5-$CH_3$ | H | |
| 2.16 | Cl | Cl | H | 3-Cl | 5-Cl | |
| 2.17 | Cl | Cl | $CH_3$ | H | H | |
| 2.18 | Cl | Cl | $CH_3$ | 3-Cl | 5-$CF_3$ | |
| 2.19 | Cl | Cl | $CH_3$ | 4-$COOCH_3$ | 6-Cl | |
| 2.20 | Cl | Cl | $CH_3$ | 5-$CH_3$ | H | |
| 2.21 | Br | Br | $CH_3$ | H | H | |
| 2.22 | Br | Br | $CH_3$ | 3-Cl | 5-$CF_3$ | |
| 2.23 | F | F | $CH_3$ | H | H | |
| 2.24 | J | J | $CH_3$ | H | H | |

Bei den beschriebenen Herstellungsverfahren können bei Verwendung von Acylhalogeniden, die zusätzlich über aktive Halogenatome verfügen, unter Salzbildung Zyklisierungen im Molekül der Endprodukte stattfinden.

Ein Beispiel dieser Art ist:

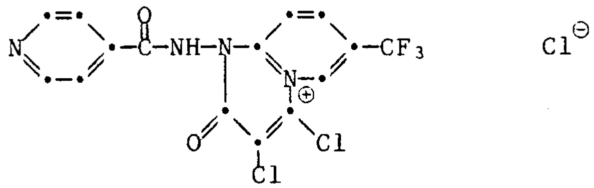

Fp. > 200°C

2. Formulierungsbeispiele für flüssige Wirkstoffe der Formel I (% = Gewichtsprozent)

2.1 Emulsions-Konzentrate

| | a) | b) | c) |
|---|---|---|---|
| Wirkstoff aus den Tabellen 1 und 2 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

2.2 Lösungen

| | a) | b) | c) | d) |
|---|---|---|---|---|
| Wirkstoff aus den Tabellen 1 und 2 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 ° C) | - | - | 94 % | - |
| (MG = Molekulargewicht) | | | | |

Die Lösungen sind zur Anwendung in Form kleinster Tropfen geeignet.

2.3 Granulate

| | a) | b) |
|---|---|---|
| Wirkstoff aus den Tabellen 1 und 2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Stäubemittel

| | a) | b) |
|---|---|---|
| Wirkstoff aus den Tabellen 1 und 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

2.5 Spritzpulver

| | a) | b) | c) |
|---|---|---|---|
| Wirkstoff aus den Tabellen 1 und 2 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

2.6 Emulsions-Konzentrat

| | |
|---|---|
| Wirkstoff aus den Tabellen 1 und 2 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.7 Stäubemittel

| | a) | b) |
|---|---|---|
| Wirkstoff aus den Tabellen 1 und 2 | 5 % | 8 % |
| Talkum | 95 % | - |
| Kaolin | - | 92 % |

Man erhält anwendungsfertige Stäubemittel, indem der Wirkstoff mit den Träger vermischt und auf einer geeigneten Mühle vermahlen wird.

2.8 Extruder Granulat

| | |
|---|---|
| Wirkstoff aus den Tabellen 1 und 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

| | |
|---|---|
| Wirkstoff aus den Tabellen 1 und 2 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | |
|---|---|
| Wirkstoff aus den Tabellen 1 und 2 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Residual protektive Wirkung

Gurkenpflanzen werden nach 2-wöchiger Anzucht mit einer aus Spritzpulver des Wirkstoffes hergestellten Spritzbrühe besprüht (Konzentration: 200 ppm).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C bis 23 °C weitergeführt.

Die Beurteilung der Schutzwirkung erfolgt aufgrund des Pilzbefalls 7-8 Tage nach der Infektion.

b) Systemische Wirkung

Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die

Inkubation wird dann bei normaler Luftfeuchtigkeit und bei 22 °C weitergeführt.

Die Beurteilung der Schutzwirkung erfolgt aufgrund des Pilzbefalls 7-8 Tage nach der Infektion.

Verbindungen aus den Tabellen 1 und 2 zeigten in den Tests (a) und (b) gute Wirkung. So reduzierten z.B. die Verbindungen 1.1, 1.4, 1.5, 1.6, 1.7, 1.10, 1.11, 1.13, 1.33, 1.72, 1.73, 1.74, 1.75, 2.1, 2.2 und 2.3 den Pilzbefall auf 0 bis 20 %. Unbehandelte aber infizierte Kontrollpflanzen wiesen dagegen einen Colletotrichum-Befall von 100 % auf.

Beispiel 3.2: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

b) Systemische Wirkung

Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Verbindungen aus den Tabellen 1 und 2 zeigten gegen Puccinia-Pilze eine gute Wirkung. So reduzierten z.B. im Test (a) die Verbindungen 1.1, 1.5, 1.7, 1.16, 1.33, 1.43, 1.74, 1.75, 2.1, 2.2 und 2.3 und im Test (b) die Verbindungen 1.1, 1.3, 1.72 und 1.73 den Pilzbefall auf 0 bis 20 %. Unbehandelte aber infizierte Kontrollpflanzen wiesen dagegen einen Puccinia-Befall von 100 % auf.

Beispiel 3.3: Wirkung gegen Phytophthora infestans auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

b) Systemische Wirkung

Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzen infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 und 2 zeigten gegen den Phytophthora-Pilz eine gute systemische Wirkung. So reduzierten z.B. im Test (a) die Verbindungen 1.1, 1.72 und 1.73 und im Test (b) die Verbindungen 1.1, 1.33, 1.71, 1.73 und 2.2 den Pilzbefall auf 0 bis 20 %. Unbehandelte aber infizierte Kontrollpflanzen wiesen dagegen einen 100 %igen Phytophthora-Befall auf.

Beispiel 3.4: Wirkung gegen Cercospora arachidicola auf Erdnusspflanzen Residual-protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1 und 2 behandelt wurden, einen stark reduzierten Cercospora-Befall.

Beispiel 3.5: Wirkung gegen Plasmopara viticola auf Reben

a) Residual-protektive Wirkung
Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 °C wird der Pilzbefall beurteilt.
b) Residual-kurative Wirkung
Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen 1 und 2 zeigten gegen Plasmopara viticola eine gute fungizide Wirkung. Unbehandelte aber infizierte Kontrollpflanzen wiesen dagegen einen 100%igen Plasmopara-Befall auf.

Beispiel 3.6: Wirkung gegen Piricularia oryzae auf Reispflanzen Residual-protektive Wirkung

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.
b) Systemische Wirkung: Zu 2-wöchigen Reispflanzen wird eine aus Spritzpulver des Wirkstoffes hergestellte Spritzbrühe gegessen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24 °C wird der Pilzbefall beurteilt.

Reispflanzen, die mit einer Spritzbrühe behandelt wurden, die als Aktivsubstanz eine Verbindung aus den Tabellen 1 und 2 enthielt, zeigten im Vergleich zu unbehandelten Kontrollpflanzen (100 % Befall) nur geringen Pilzbefall. So reduzierten z.B. im Test (a) die Verbindungen 1.7, 1.71, 1.72, 1.73 und 2.3 und im Test (b) die Verbindungen 1.12, 1.16, 1.43, 1.71, 1.72, 1.73, 1.74 und 2.3 den Pilzbefall auf 5 bis 20 %.

Beispiel 3.7: Immunisierende Wirkung gegen Colletotrichum lagenarium auf Cucumis sativus L.

Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchigkeit und bei 22 ° bis 23 °C weitergeführt.

Die Beurteilung der Schutzwirkung erfolgt aufgrund des Pilzbefalls 7-8 Tage nach der Infektion.

Unbehandelte aber infizierte Kontrollpflanzen wiesen in dem Test einen Pilzbefall von 100 % auf.

Verbindungen aus den Tabellen 1 und 2 bewirkten eine gute Immunisierung gegen Colletotrichum.

Beispiel 3.8: Immunisierende Wirkung gegen den Tabakmosaik-Virus auf Tabak

8 Wochen alte Tabakpflanzen werden mit einer formulierten Lösung des Wirkstoffes besprüht (Konzentration: 200 ppm) oder injiziert (Konzentration: 200 ppm). Nach 4 Tagen werden die Pflanzen mit einer Suspension des Tabakmosaik-Virus (0.5 $\mu$g/ml + Carborundum) mechanisch inokuliert und bei einer Temperatur von 20 ° - 22 °C inkubiert.

Die Beurteilung der Schutzwirkung erfolgt aufgrund der Anzahl und Grösse der Lokalläsionen 7 Tage nach der Inokulation.

Verbindungen aus den Tabellen 1 und 2 bewirkten eine gute Immunisierung gegen den Tabakmosaik-Virus, wobei z.B. die Verbindungen 1.1 und 1.3 herausragten. Infizierte aber unbehandelte Kontrollpflanzen wiesen dagegen Läsionen von 100 % auf.

Beispiel 3.9: Wirkung gegen Pseudomonas lachrymans auf Cucumis sativus L.

a) Residual-protektive Wirkung

Gurkenpflanzen werden nach 2-wöchiger Anzucht mit einer aus Spritzpulver des Wirkstoffes hergestellten Spritzbrühe besprüht
(Konzentration: 20 ppm).

Nach 1 Woche werden die Pflanzen mit einer Bakteriensuspension ($10^8$ Bakterien/ml infiziert und für 7 Tage bei hoher Luftfeuchtigkeit und einer Temperatur von 23 °C inkubiert.

Die Beurteilung der Schutzwirkung erfolgt aufgrund des Bakterienbefalls 7-8 Tage nach der Infektion.

b) Systemische Wirkung

Gurkenpflanzen werden nach 2-wöchiger Anzucht mit einer aus Spritzpulver des Wirkstoffes hergestellten Spritzbrühe durch Bodenapplikation behandelt (Konzentrationen: 60, 20, 6, 2 ppm bezogen auf das Bodenvolumen).

Nach 1 Woche werden die Pflanzen mit einer Bakteriensuspension ($10^8$ Bakterien/ml) infiziert und für 7 Tage bei hoher Luftfeuchtigkeit und einer Temperatur von 23 °C inkubiert.

Die Beurteilung der Schutzwirkung erfolgt aufgrund des Bakterienbefalls 7 - 8 Tage nach der Infektion.

Verbindungen aus den Tabellen 1 und 2 zeigten eine gute Schutzwirkung gegen Pseudomonasbefall. So reduzierte z.B. in den Tests (a) und (b) die Verbindung 1.1 den Bakterienbefall auf 0 bis 10 %. Unbehandelte aber infizierte Kontrollpflanzen wiesen dagegen einen 100 %igen Pseudomonas-Befall auf.

Beispiel 3.10: Wirkung gegen Xanthomonas oryzae auf Reis (Oryza sativa)

a) Residual-protektive Wirkung

Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Systemische Wirkung

Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 und 2 zeigten eine gute Wirkung gegen Xanthomonas oryzae. So reduzierten z.B. im Test (a) die Verbindungen 1.6, 1.33, 2.1 und 2.2 und im Test (b) 1.1, 1.3, 1.4, 1.8, 1.10, 1.11, 1.33, 1.71, 1.73, 1.75, 2.1, 2.2 und 2.3 den Pilzbefall auf 0 bis 20 %. Unbehandelte jedoch infizierte Kontrollpflanzen wiesen dagegen einen Krankheitsbefall von 100 % auf.

Beispiel 3.11: Wirkung gegen Xanthomonas vesicatoria auf Paprika (Capsicum annuum)

a) Residual-protektive Wirkung

Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Systemische Wirkung

Paprikapflanzen der Sorte "California Wonder" werden nach 3-wöchiger Anzucht im Gewächshaus mit

einer Suspension der Prüfsubstanz begossen (0,006 % Aktivsubstanz bezogen auf das Erdvolumen). Drei Tage nach dieser Behandlung werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 und 2 zeigten eine gute Wirkung gegen Xanthomonas vesicatoria. So reduzierten z.B. im Test (a) die Verbindungen 1.1, 1.3, 1.33, 2.1 und 2.2 und im Test (b) 1.1, 1.3, 1.4, 1.8, 1.10, 1.11, 1.33, 1.71, 1.73, 2.1 und 2.2 den Bakterienbefall auf 0 bis 20 %. Unbehandelte jedoch infizierte Kontrollpflanzen wiesen dagegen einen Krankheitsbefall von 100 % auf.

Beispiel 3.12: Beizwirkung gegen Fusarium nivale an Roggen

Natürlich mit Fusarium nivale infizierter Roggen der Sorte Tetrahell wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Der infizierte und behandelte Roggen wird im Oktober im Freiland mittels einer Sämaschine auf Parzellen von 3 m Länge und 6 Saatreihen ausgesät, mit 3 Wiederholungen pro Versuchsprodukt.

Bis zur Befallsauswertung wird die Versuchspflanzung unter normalen Feldbedingungen kultiviert (vorzugsweise in einer Region mit geschlossener Schneedecke während der Wintermonate).

Zur Ermittlung der Wirkstoffaktivität wird im Frühjahr unmittelbar nach der Schneeschmelze, der prozentuale Anteil mit Fusarium befallener Pflanzen ausgezählt.

Verbindungen aus den Tabellen 1 und 2 zeigten eine gute Wirkung gegen Fusarium. Unbehandelte jedoch infizierte Kontrollpflanzen wiesen einen Krankheitsbefall von 100 % auf.

Beispiel 3.13: Beizwirkung gegen Helminthospora gramineum an Gerste

Natürlich mit Helminthosporium gramineum infizierte Wintergerste der Sorte "Cl" wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil mit Helminthospora befallener Halme ausgezählt.

Verbindungen aus den Tabellen 1 und 2 zeigten eine gute Wirkung gegen Helminthospora. Unbehandelte jedoch infizierte Kontrollpflanzen wiesen einen Krankheitsbefall von 100 % auf.

Beispiel 3.14: Beizwirkung gegen Ustilago nuda an Gerste

Natürlich mit Ustilago nuda infizierte Wintergerste der Sorte "RM1" wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität wird während der Blüte der prozentuale Anteil Ustilago befallener Aehren ausgezählt.

Verbindungen aus den Tabellen 1 und 2 zeigten eine gute Wirkung gegen Ustilago. Unbehandelte jedoch infizierte Kontrollpflanzen wiesen einen Krankheitsbefall von 100 % auf.

Beispiel 3.15: Beizwirkung gegen Colletotrichum lagenarium an Cucumis sativus L.

Gurkensamen werden mit einer Lösung des Wirkstoffes gebeizt (Konzentration: 180 g/100 kg Samen). Die Samen werden ausgesät. Nach 4 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22° bis 23 °C weitergeführt. Die Beurteilung der Schutzwirkung erfolgt aufgrund des Pilzbefalls 7 - 8 Tage nach der Infektion.

Verbindungen aus den Tabellen 1 und 2 zeigten eine gute Wirkung gegen Colletotrichum. So reduzierten z.B. die Verbindungen 1.1 und 2.2 den Pilzbefall auf 0 bis 20 %. Infizierte Kontrollpflanzen, deren Samen nicht behandelt wurden, wiesen dagegen einen Pilzbefall von 100 % auf.

Beispiel 3.16: Residual-protektive Wirkung gegen Venturia inaequalis auf Apfeltrieben

Apfelstecklinge mit 10 - 20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relativer Luftfeuchtigkeit inkubiert und während 10 weiteren Tagen in einem Gewächshaus bei 20 - 24 °C aufgestellt. Der Schorfbefall wird 15 Tage nach der Infektion beurteilt.

Verbindungen aus den Tabellen 1 und 2 zeigten gute Wirksamkeit gegen Venturia. So reduzierten z.B. die Verbindungen 1.71 und 2.2 den Schorfbefall auf 5 bis 20 %. Unbehandelte jedoch infizierte Triebe wiesen dagegen einen 100 %igen Venturia-Befall auf.

Beispiel 3.17: Wirkung gegen Cercospora nicotianae auf Tabak

8 Wochen alte Tabakpflanzen werden mit einer formulierten Lösung des Wirkstoffs injiziert (Konzentration: 200 ppm). In einem Zeitraum von 2 Stunden bis 4 Tagen nach der Behandlung werden die Pflanzen mit einer Sporensuspension von Cercospora nicotianae ($10^5$ Sporen/ml) besprüht und für 5 Tage bei hoher Luftfeuchtigkeit und einer Temperatur von 22° - 25°C inkubiert. Anschliessend wird die Inkubation bei normaler Luftfeuchtigkeit und 20° - 22°C fortgesetzt. Die Beurteilung der Symptome erfolgt dann aufgrund des Pilzbefalls 12 bis 14 Tage nach der Infektion.

Verbindungen aus den Tabellen 1 und 2 zeigten eine gute Wirkung gegen Cercospora. So reduzierte z.B. die Verbindung 1.1 den Pilzbefall auf 0 bis 20 %. Infizierte Kontrollpflanzen wiesen einen Pilzbefall von 100 % auf.

**Patentansprüche**

**Patentansprüche für folgende Vertragsstaaten : AT, BE, CH, DE, FR, GB, GR, IT, LI, LU, NL**

**1.** Verbindungen der Formel I

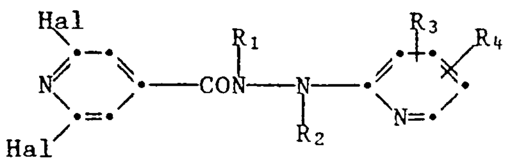

(I)

in welcher bedeuten:

- Hal Halogen;
- $R_1$ Wasserstoff, Methyl oder den Rest $COR_5$
- $R_2$ Wasserstoff oder einen der Reste $COR_5$ oder $CO\text{-}Z\text{-}R_6$;
- $R_3$ Wasserstoff, Halogen, Trifluormethyl, Trichlormethyl, COOH, $COOCH_3$, OH oder Nitro;
- $R_4$ Wasserstoff, Halogen, Methoxy oder Methyl;
- $R_5$ $C_1$-$C_6$-Alkyl, ein- oder mehrfach mit Halogen substituiertes $C_1$-$C_6$-Alkyl, durch Sauerstoff oder Schwefel unterbrochenes $C_1$-$C_6$-Alkyl, ein- oder mehrfach mit Halogen substituiertes und durch Sauerstoff oder Schwefel unterbrochenes $C_1$-$C_6$-Alkyl, $C_2$-$C_4$-Alkenyl, ein- oder mehrfach mit Halogen substituiertes $C_2$-$C_4$-Alkenyl, Phenyl, durch Halogen, Methyl, Trifluormethyl oder Trichlormethyl substituiertes Phenyl, Benzyl, durch Halogen, Methyl, Trifluormethyl oder Trichlormethyl substituiertes Benzyl, ein 5- oder 6-gliedriger Heterocyclus mit Stickstoff, Sauerstoff oder Schwefel als Heteroatome, ein 3- bis 6-gliedriges Cycloalkyl oder ein einfach- oder mehrfach durch Halogen oder Methyl substituiertes Cycloalkyl;
- $R_6$ $C_1$-$C_5$-Alkyl, Phenyl oder falls Z die CO-Gruppe darstellt $OC_1$-$C_2$-Alkyl; und
- Z Sauerstoff, Schwefel oder die CO-Gruppe; mit Ausnahme von 1(-Pyridin-2'-yl)-2-(2,6-dichlorisonicotinoyl)-hydrazin.

**2.** Verbindungen der Formel I gemäss Anspruch 1, in welchen

Hal Chlor oder Brom bedeutet;

$R_1$ Wasserstoff ist;

$R_2$ Wasserstoff oder eine der Gruppen $COCH_3$, $COC_2H_5$ oder

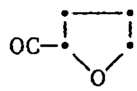

darstellt; und

$R_3$ und $R_4$ unabhängig voneinander für Wasserstoff, Halogen oder Trifluormethyl stehen.

**3.** Verbindungen der Formel I gemäss Anspruch 1, in welchen

Hal Chlor bedeutet;

$R_1$ Wasserstoff ist;

$R_2$ Wasserstoff darstellt; und

$R_3$ und $R_4$ unabhängig voneinander für Wasserstoff, Halogen oder Trifluormethyl stehen.

**4.** Verbindungen der Formel I gemäss Anspruch 1, in welchen

Hal Chlor bedeutet;

$R_1$ Wasserstoff ist;

$R_2$ eine der Gruppen $COCH_3$, $COC_2H_5$ oder

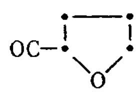

darstellt; und

$R_3$ und $R_4$ unabhängig voneinander für Wasserstoff, Halogen oder Trifluormethyl stehen.

**5.** Verbindungen der Formel I gemäss Anspruch 1, in welchen

Hal Chlor bedeutet;

$R_1$ Wasserstoff ist;

$R_2$ Wasserstoff darstellt;

$R_3$ für Wasserstoff oder 3-Chlor steht; und

$R_4$ für Wasserstoff oder 5-Trifluormethyl steht.

**6.** Verbindungen der Formel I gemäss Anspruch 1, in welchen

Hal Chlor bedeutet;

$R_1$ Wasserstoff ist;

$R_2$ eine der Gruppen $COCH_3$, $COC_2H_5$ oder

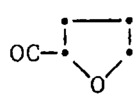

darstellt;

$R_3$ für Wasserstoff oder 3-Chlor steht; und

$R_4$ für Wasserstoff oder 5-Trifluormethyl steht.

**7.** Eine Verbindung der Formel I nach Anspruch 1, ausgewählt aus der Gruppe

1-Acetyl-1-pyridin-2'-yl)-2-(2,6-dichlorisonicotinoyl)-hydrazin;

1-Propionyl-1-pyridin-2'-yl)-2-(2,6-dichlorisonicotinoyl)-hydrazin;

1-Tetrahydrofuroyl-1-pyridin-2'-yl)-2-(2,6-dichlorisonicotinoyl)-hydrazin;

1-(3'-Chlor-5'-trifluormethylpyridin-2'-yl)-(2,6-dichlorisonicotinoyl)-hydrazin;

1-Methyl-1-(3'-chlor-5'-trifluormethylpyridin-2'-yl)-(2,6-dichlorisonicotinoyl)-hydrazin;
1-Ethyl-1-(3'-chlor-5'-trifluormethylpyridin-2'-yl)-(2,6-dichlorisonicotinoyl)-hydrazin.

**8.** Verfahren zur Herstellung der Verbindungen der Formel I gemäss Anspruch 1, dadurch gekennzeichnet, dass man

a) 2-Hydrazino-pyridin-Derivate der Formel II

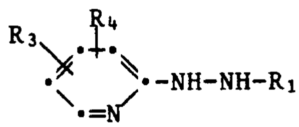

(II)

mit 2,6-Dihaloisonicotinoyl-Derivaten der Formel III

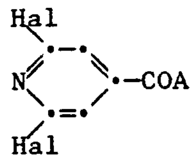

(III)

in einem inerten Lösungsmittel oder

b) 2,6-Dihaloisonicotinsäurehydrazid-Derivate der Formel IV

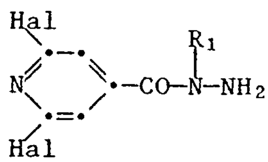

(IV)

mit substituierten 2-Halopyridin-Derivaten der Formel V

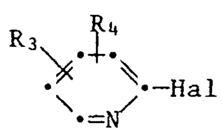

(V)

in einem inerten Lösungsmittel gegebenenfalls in Gegenwart eines Katalysators umsetzt, und anschliessend

c) die erhaltenen Verbindungen der Formel Ia

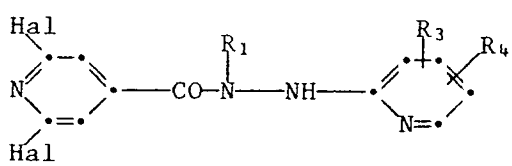

(Ia)

mit Verbindungen der Formel (VI)

$R_2CO\text{-}Y$ (VI)

in einem inerten Lösungsmittel gegebenenfalls in Gegenwart einer Base reagieren lässt, wobei A Halogen, O-$C_1$-$C_4$-Alkyl oder S-$C_1$-$C_4$-Alkyl darstellt und Y Halogen oder O-$C_1$-$C_4$-Alkyl bedeutet und Hal und $R_1$ bis $R_4$ die unter Formel I angegebenen Bedeutungen besitzen.

9. Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung der Formel I gemäss Anspruch 1 einschliesslich von 1-(Pyridin-2'-yl)-2-(2,6-dichlorisonicotinoyl)-hydrazin enthält.

10. Mittel gemäss Anspruch 9, dadurch gekennzeichnet, dass es als aktive Komponente mindestens eine Verbindung der Formel I gemäss der Ansprüche 2 - 6 enthält.

11. Mittel gemäss Anspruch 9, dadurch gekennzeichnet, dass es als aktive Komponente mindestens eine Verbindung der Formel I gemäss Anspruch 7 enthält.

12. Verfahren zur Herstellung eines wie in Anspruch 9 beanspruchten agrochemischen Mittels, dadurch gekennzeichnet, dass man mindestens eine gemäss Anspruch 1 definierte Verbindung der Formel I einschliesslich von 1-(Pyridin-2'-yl)-2-(2,6-dichlorisonicotinoyl)-hydrazin mit geeigneten festen oder flüssigen Träger- und Hilfsstoffen innig vermischt.

13. Verwendung von Verbindungen der Formel I einschliesslich von 1-(Pyridin-2'-yl)-2-(2,6-dichlorisonicotinoyl)-hydrazin gemäss Anspruch 1 zum Schutz von Pflanzen gegen den Befall durch phytopathogene Mikroorganismen.

14. Verwendung von Verbindungen der Formel I gemäss einem der Ansprüche 2 bis 7 zum Schutz von Pflanzen gegen den Befall durch phytopathogene Mikroorganismen.

15. Verfahren zum Schutz von Pflanzen gegen den Befall durch phytopathogene Mikroorganismen, dadurch gekennzeichnet, dass man als Wirkstoff eine Verbindung der Formel I einschliesslich von 1-(Pyridin-2'-yl)-2-(2,6-dichlorisonicotinoyl)-hydrazin gemäss Anspruch 1 auf die Pflanze oder deren Standort appliziert.

16. Verfahren zum Schutz von Pflanzen gegen den Befall durch phytopathogene Mikroorganismen, dadurch gekennzeichnet, dass man als Wirkstoff eine Verbindung der Formel I gemäss einem der Ansprüche 2 bis 7 auf die Pflanze oder deren Standort appliziert.

17. Verfahren zur Immunisierung von Pflanzen gegen den Befall durch phytopathogene Mikroorganismen, dadurch gekennzeichnet, dass man als Wirkstoff eine Verbindung der Formel I einschliesslich von 1-(Pyridin-2'-yl)-2-(2,6-dichlorisonicotinoyl)-hydrazin gemäss Anspruch 1 auf die Pflanze oder deren Standort appliziert.

18. Verfahren zur Immunisierung von Pflanzen gegen den Befall durch phytopathogene Mikroorganismen, dadurch gekennzeichnet, dass man als Wirkstoff eine Verbindung der Formel I gemäss einem der Ansprüche 2 bis 7 auf die Pflanze oder deren Standort appliziert.

19. Verfahren gemäss den Ansprüchen 15 und 17, dadurch gekennzeichnet, dass es sich bei den phytopathogenen Mikroorganismen um Pilzorganismen handelt.

20. Verfahren gemäss Anspruch 19, dadurch gekennzeichnet, dass es Pilzorganismen aus den Klassen Ascomycetes, Basidiomycetes oder Fungi imperfecti sind.

21. Verfahren gemäss den Ansprüchen 15 und 17, dadurch gekennzeichnet, dass es sich bei den phytopathogenen Mikroorganismen um Bakterien handelt.

22. Verfahren gemäss den Ansprüchen 15 und 17, dadurch gekennzeichnet, dass es sich bei den phytopathogenen Mikroorganismen um Viren handelt.

**Patentansprüche für folgenden Vertragsstaat : ES**

**1.** Verfahren zur Herstellung von Verbindungen der Formel I

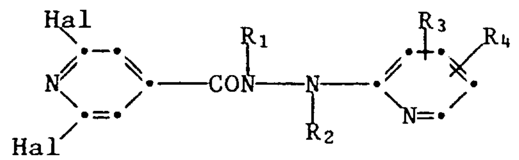

(I)

in welcher bedeuten:

Hal Halogen;

$R_1$ Wasserstoff, Methyl oder den Rest $COR_5$

$R_2$ Wasserstoff oder einen der Reste $COR_5$ oder CO-Z-$R_6$;

$R_3$ Wasserstoff, Halogen, Trifluormethyl, Trichlormethyl, COOH, $COOCH_3$, OH oder Nitro;

$R_4$ Wasserstoff, Halogen, Methoxy oder Methyl;

$R_5$ $C_1$-$C_6$-Alkyl, ein- oder mehrfach mit Halogen substituiertes $C_1$-$C_6$-Alkyl, durch Sauerstoff oder Schwefel unterbrochenes $C_1$-$C_6$-Alkyl, ein- oder mehrfahc mit Halogen substituiertes und durch Sauerstoff oder Schwefel unterbrochenes $C_1$-$C_6$-Alkyl, $C_2$-$C_4$-Alkenyl, ein- oder mehrfach mit Halogen substituiertes $C_2$-$C_4$-Alkenyl, Phenyl, durch Halogen, Methyl. Trifluormethyl oder Trichlormethyl substituiertes Phenyl, Benzyl, durch Halogen, Methyl, Trifluormethyl oder Trichlormethyl substituiertes Benzyl, ein 5- oder 6-gliedriger Heterocyclus mit Stickstoff, Sauerstoff oder Schwefel als Heteroatome, ein 3- bis 6-gliedriges Cycloalkyl oder ein einfach- oder mehrfach durch Halogen oder Methyl substituiertes Cycloalkyl;

$R_6$ $C_1$-$C_5$-Alkyl, Phenyl oder falls Z die CO-Gruppe darstellt $OC_1$-$C_2$-Alkyl; und

Z Sauerstoff, Schwefel oder die CO-Gruppe; mit Ausnahme von 1(-Pyridin-2'-yl)-2-(2,6-dichlorisonicotinoyl)-hydrazin, dadurch gekennzeichnet, dass man

a) 2-Hydrazino-pyridin-Derivate der Formel II

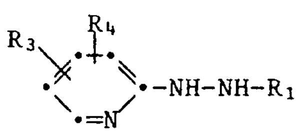

(II)

mit 2,6-Dihaloisonicotinoyl-Derivaten der Formel III

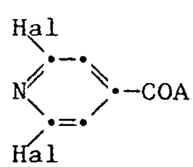

(III)

in einem inerten Lösungsmittel oder

b) 2,6-Dihaloisonicotinsäurehydrazid-Derivate der Formel IV

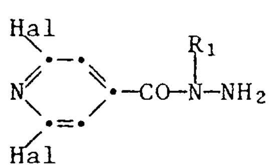

(IV)

mit substituierten 2-Halopyridin-Derivaten der Formel V

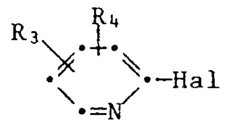

(V)

in einem inerten Lösungsmittel gegebenenfalls in Gegenwart eines Katalysators umsetzt, und anschliessend

c) die erhaltenen Verbindungen der Formel Ia

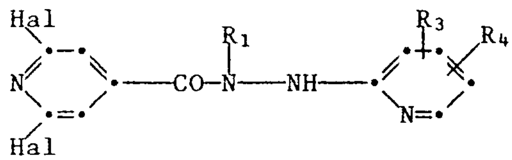

(Ia)

mit Verbindungen der Formel VI

$R_2CO$-Y (VI)

in einem inerten Lösungsmittel gegebenenfalls in Gegenwart einer Base reagieren lässt, wobei A Halogen, O-$C_1$-$C_4$-Alkyl oder S-$C_1$-$C_4$-Alkyl darstellt und Y Halogen oder O-$C_1$-$C_4$-Alkyl bedeutet und Hal und $R_1$ bis $R_4$ die unter Formel I angegebenen Bedeutungen besitzen.

**2.** Verfahren gemäss Anspruch 1 zur Herstellung der Verbindungen der Formel I, in welchen

| | |
|---|---|
| Hal | Chlor oder Brom bedeutet; |
| $R_1$ | Wasserstoff ist; |
| $R_2$ | Wasserstoff oder eine der Gruppen $COCH_3$, $COC_2H_5$ oder |

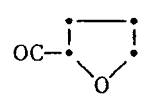

darstellt; und

| | |
|---|---|
| $R_3$ und $R_4$ | unabhängig voneinander für Wasserstoff, Halogen oder Trifluormethyl stehen. |

**3.** Verfahren gemäss Anspruch 1 zur Herstellung der Verbindungen der Formel I, in welchen

| | |
|---|---|
| Hal | Chlor bedeutet; |
| $R_1$ | Wasserstoff ist; |
| $R_2$ | Wasserstoff, darstellt; und |
| $R_3$ und $R_4$ | unabhängig voneinander für Wasserstoff, Halogen oder Trifluormethyl stehen. |

**4.** Verfahren gemäss Anspruch 1 zur Herstellung der Verbindungen der Formel I, in welchen

| | |
|---|---|
| Hal | Chlor bedeutet; |
| $R_1$ | Wasserstoff ist; |
| $R_2$ | eine der Gruppen $COCH_3$, $COC_2H_5$ oder |

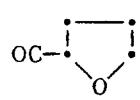

darstellt; und

| | |
|---|---|
| $R_3$ und $R_4$ | unabhängig voneinander für Wasserstoff, Halogen oder Trifluormethyl stehen. |

**5.** Verfahren gemäss Anspruch 1 zur Herstellung der Verbindungen der Formel I, in welchen
Hal Chlor bedeutet;
$R_1$ Wasserstoff ist;
$R_2$ Wasserstoff darstellt;
$R_3$ für Wasserstoff oder 3-Chlor steht; und
$R_4$ für Wasserstoff oder 5-Trifluormethyl steht.

**6.** Verfahren gemäss Anspruch 1 zur Herstellung der Verbindungen der Formel I, in welchen
Hal Chlor bedeutet;
$R_1$ Wasserstoff ist;
$R_2$ eine der Gruppen $COCH_3$, $COC_2H_5$ oder

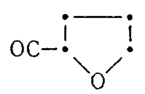

darstellt;
$R_3$ für Wasserstoff oder 3-Chlor steht; und
$R_4$ für Wasserstoff oder 5-Trifluormethyl steht.

**7.** Verfahren gemäss Anspruch 1 zur Herstellung einer Verbindung der Formel I, ausgewählt aus der Gruppe
1-Acetyl-1-pyridin-2'-yl)-2-(2,6-dichlorisonicotinoyl)-hydrazin;
1-Propionyl-1-pyridin-2'-yl)-2-(2,6-dichlorisonicotinoyl)-hydrazin;
1-Tetrahydrofuroyl-1-pyridin-2'-yl)-2-(2,6-dichlorisonicotinoyl)-hydrazin;
1-(3'-Chlor-5'-trifluormethylpyridin-2'-yl)-(2,6-dichlorisonicotinoyl)-hydrazin;
1-Methyl-1-(3'-chlor-5'-trifluormethylpyridin-2'-yl)-(2,6-dichlorisonicotinoyl)-hydrazin;
1-Ethyl-1-(3'-chlor-5'-trifluormethylpyridin-2'-yl)-(2,6-dichlorisonicotinoyl)-hydrazin.

**8.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung der Formel I gemäss Anspruch 1 einschliesslich von 1-(Pyridin-2'-yl)-2-(2,6-dichlorisonicotinoyl)-hydrazin enthält.

**9.** Mittel gemäss Anspruch 8, dadurch gekennzeichnet, dass es als aktive Komponente mindestens eine Verbindung der Formel I gemäss der Ansprüche 2 - 6 enthält.

**10.** Mittel gemäss Anspruch 8, dadurch gekennzeichnet, dass es als aktive Komponente mindestens eine Verbindung der Formel I gemäss Anspruch 7 enthält.

**11.** Verfahren zur Herstellung eines wie in Anspruch 8 beanspruchten agrochemischen Mittels, dadurch gekennzeichnet, dass man mindestens eine gemäss Anspruch 1 definierte Verbindung der Formel I einschliesslich von 1-(Pyridin-2'-yl)-2-(2,6-dichlorisonicotinoyl)-hydrazin mit geeigneten festen oder flüssigen Träger- und Hilfsstoffen innig vermischt.

**12.** Verfahren zum Schutz von Pflanzen gegen den Befall durch phytopathogene Mikroorganismen, dadurch gekennzeichnet, dass man als Wirkstoff eine Verbindung der Formel I einschliesslich von 1-(Pyridin-2'-yl)-2-(2,6-dichlorisonicotinoyl)-hydrazin gemäss Anspruch 1 auf die Pflanze oder deren Standort appliziert.

**13.** Verfahren zum Schutz von Pflanzen gegen den Befall durch phytopathogene Mikroorganismen, dadurch gekennzeichnet, dass man als Wirkstoff eine Verbindung der Formel I gemäss einem der Ansprüche 2 bis 7 auf die Pflanze oder deren Standort appliziert.

**14.** Verfahren zur Immunisierung von Pflanzen gegen den Befall durch phytopathogene Mikroorganismen, dadurch gekennzeichnet, dass man als Wirkstoff eine Verbindung der Formel I einschliesslich von 1-(Pyridin-2'-yl)-2-(2,6-dichlorisonicotinoyl)-hydrazin gemäss Anspruch 1 auf die Pflanze oder deren

Standort appliziert.

**15.** Verfahren zur Immunisierung von Pflanzen gegen den Befall durch phytopathogene Mikroorganismen, dadurch gekennzeichnet, dass man als Wirkstoff eine Verbindung der Formel I gemäss einem der Ansprüche 2 bis 7 auf die Pflanze oder deren Standort appliziert.

**16.** Verfahren gemäss den Ansprüchen 12 und 14, dadurch gekennzeichnet, dass es sich bei den phytopathogenen Mikroorganismen um Pilzorganismen handelt.

**17.** Verfahren gemäss Anspruch 16, dadurch gekennzeichnet, dass es Pilzorganismen aus den Klassen Ascomycetes, Basidiomycetes oder Fungi imperfecti sind.

**18.** Verfahren gemäss den Ansprüchen 12 und 14, dadurch gekennzeichnet, dass es sich bei den phytopathogenen Mikroorganismen um Bakterien handelt.

**19.** Verfahren gemäss den Ansprüchen 12 und 14, dadurch gekennzeichnet, dass es sich bei den phytopathogenen Mikroorganismen um Viren handelt.

**20.** Verwendung von Verbindungen der Formel I einschliesslich von 1-(Pyridin-2'-yl)-2-(2,6-dichlorisonicotinoyl)-hydrazin gemäss Anspruch 1 zum Schutz von Pflanzen gegen den Befall durch phytopathogene Mikroorganismen.

**21.** Verwendung von Verbindungen der Formel I gemäss einem der Ansprüche 2 bis 7 zum Schutz von Pflanzen gegen den Befall durch phytopathogene Mikroorganismen.

## Claims

**Claims for the following Contracting States : AT, BE, CH, DE, FR, GB, GR, IT, LI, LU, NL**

**1.** A compound of formula I

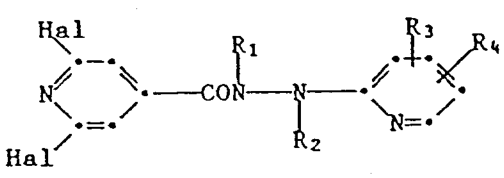

(I)

wherein

Hal is halogen;
$R_1$ is hydrogen, methyl or the radical $COR_5$;
$R_2$ is hydrogen or a radical $COR_5$ or $CO\text{-}Z\text{-}R_6$;
$R_3$ is hydrogen, halogen, trifluoromethyl, trichloromethyl, COOH, $COOCH_3$, OH or nitro;
$R_4$ is hydrogen, halogen, methoxy or methyl;
$R_5$ is $C_1$-$C_6$alkyl, unsubstituted or substituted by one or more halogen atoms, $C_1$-$C_6$alkyl which is interrupted by oxygen or sulphur, $C_1$-$C_6$alkyl which is substituted by one or more halogen atoms and interrupted by oxygen or sulphur, $C_2$-$C_4$alkenyl, unsubstituted or substituted by one or more halogen atoms, phenyl, benzyl, or phenyl or benzyl each substituted by halogen, methyl, trifluoromethyl or trichloromethyl, a 5- or 6-membered heterocycle which contains nitrogen, oxygen or sulphur as hetero atoms, a 3- to 6-membered cycloalkyl radical, or a cycloalkyl radical which is substituted by one or more halogen atoms or methyl groups;
$R_6$ is $C_1$-$C_5$alkyl, phenyl or, if Z is the CO group, is O-alkyl containing 1 or 2 carbon atoms; and
Z is oxygen, sulphur or the CO group;

with the exception of 1-(pyridin-2'-yl)-2-(2,6-dichloroisonicotinoyl)hydrazine.

**2.** A compound of formula I according to claim 1, wherein

Hal is chlorine or bromine;
$R_1$ is hydrogen;
$R_2$ is hydrogen or a group $COCH_3$, $COC_2H_5$ or

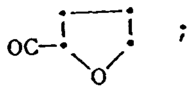

and

$R_3$ and $R_4$ are each independently of the other hydrogen, halogen or trifluoromethyl.

**3.** A compound of formula I according to claim 1, wherein

Hal is chlorine;
$R_1$ is hydrogen;
$R_2$ is hydrogen; and
$R_3$ and $R_4$ are each independently of the other hydrogen, halogen or trifluoromethyl.

**4.** A compound of formula I according to claim 1, wherein

Hal is chlorine;
$R_1$ is hydrogen;
$R_2$ is a group $COCH_3$, $COC_2H_5$ or

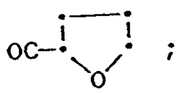

and

$R_3$ and $R_4$ are each independently of the other hydrogen, halogen or trifluoromethyl.

**5.** A compound of formula I according to claim 1, wherein

Hal is chlorine;
$R_1$ is hydrogen;
$R_2$ is hydrogen;
$R_3$ is hydrogen or 3-chloro; and
$R_4$ is hydrogen or 5-trifluoromethyl.

**6.** A compound of formula I according to claim 1, wherein

Hal is chlorine;
$R_1$ is hydrogen;
$R_2$ is a group $COCH_3$, $COC_2H_5$ or

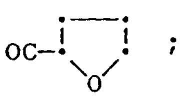

$R_3$ is hydrogen or 3-chloro; and
$R_4$ is hydrogen or 5-trifluoromethyl.

**7.** A compound of formula I according to claim 1, selected from the group

1-acetyl-1-pyridin-2'-yl)-2-(2,6-dichloroisonicotinoyl)-hydrazine;
1-propionyl-1-pyridin-2'-yl)-2-(2,6-dichloroisonicotinoyl)hydrazine;
1-tetrahydrofuroyl-1-pyridin-2'-yl)-2-(2,6-dichloroisonicotinoyl)hydrazine;
1-(3'-chloro-5'-trifluoromethylpyridin-2'-yl)-(2,6-dichloroisonicotinoyl)hydrazine;
1-methyl-1-(3'-chloro-5'-trifluoromethylpyridin-2'-yl)-(2,6-dichloroisonicotinoyl)hydrazine;
1-ethyl-1-(3'-chloro-5'-trifluoromethylpyridin-2'-yl)-(2,6-dichloroisonicotinoyl)hydrazine.

**8.** A process for the preparation of a compound of formula I according to claim 1, which comprises

a) reacting a 2-hydrazinopyridine derivative of formula II

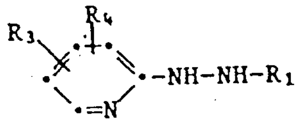

(II)

with a 2,6-dihaloisonicotinoyl derivative of formula III

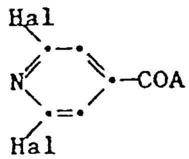

(III)

in an inert solvent, or
b) reacting a 2,6-dihaloisonicotinoyl hydrazide derivative of formula IV

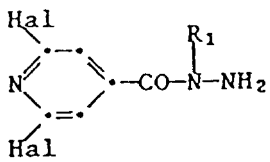

(IV)

with a substituted 2-halopyridine derivative of formula V

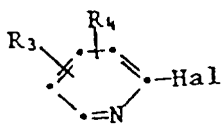

(V)

in an inert solvent and with or without a catalyst, and subsequently
c) reacting the resultant compound of formula Ia

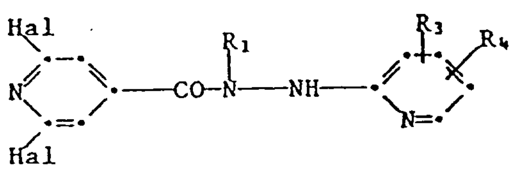

(Ia)

with a compound of formula VI

$R_2CO\text{-}Y$ (VI)

in an inert solvent and in the absence or presence of a base, in which formulae A is halogen, O-alkyl having from 1 to 4 carbon atoms or S-alkyl having from 1 to 4 carbon atoms, Y is halogen or O-alkyl having from 1 to 4 carbon atoms and Hal and $R_1$ to $R_4$ are as defined for formula I.

**9.** A composition for protecting plants against attack by microorganisms, which comprises as active component at least one compound of formula I as claimed in claim 1, including 1-(pyridin-2'-yl)-2-(2,6-dichloroisonicotinoyl)hydrazine, together with conventional carriers and adjuvants.

**10.** A composition according to claim 9, which comprises as active component at least one compound of formula I as claimed in any one of claims 2 to 6.

**11.** A composition according to claim 9, which comprises as active component at least one compound of formula I as claimed in claim 7.

**12.** A process for the preparation of an agrochemical composition as claimed in claim 9, which comprises intimately mixing at least one compound of formula I as defined in claim 1, including 1-(pyridin-2'-yl)-2-(2,6-dichloroisonicotinoyl)hydrazine, with suitable solid or liquid carriers and adjuvants.

**13.** The use of a compound of formula I, including 1-(pyridin-2'-yl)-2-(2,6-dichloroisonicotinoyl)hydrazine, as claimed in claim 1, for the protection of plants against attack by phytopathogenic microorganisms.

**14.** The use of a compound of formula I as claimed in any one of claims 2 to 7 for the protection of plants against attack by phytopathogenic microorganisms.

**15.** A method of protecting plants against attack by phytopathogenic microorganisms, which comprises applying as active ingredient to said plants or to the locus thereof a compound of formula I, including 1-(pyridin-2'-yl)-2-(2,6-dichloroisonicotinoyl)hydrazine, as claimed in claim 1.

**16.** A method of protecting plants against attack by phytopathogenic microorganisms, which comprises applying as active ingredient to said plants or to the locus thereof a compound of formula I as claimed in any one of claims 2 to 7.

**17.** A method of immunising plants against attack by phytopathogenic microorganisms, which comprises applying as active ingredient to said plants or to the locus thereof a compound of formula I, including 1-(pyridin-2'-yl)-2-(2,6-dichloroisonicotinoyl)hydrazine, as claimed in claim 1.

**18.** A method of immunising plants against attack by phytopathogenic microorganisms, which comprises applying as active ingredient to said plants or to the locus thereof a compound of formula I as claimed in any one of claims 2 to 7.

**19.** A method according to either claim 15 or claim 17, wherein the phytopathogenic microorganisms are fungi.

**20.** A method according to claim 19, wherein the fungi are from the classes Ascomycetes, Basidiomycetes or Fungi imperfecti.

**21.** A method according to either claim 15 or claim 17, wherein the phytopathogenic microorganisms are bacteria.

**22.** A method according to either claim 15 or claim 17, wherein the phytopathogenic microorganisms are viruses.

**Claims for the following Contracting State : ES**

**1.** A process for the preparation of a compound of formula I

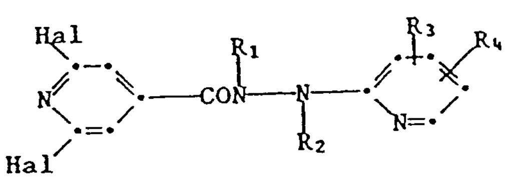

(I)

wherein

Hal is halogen;

$R_1$ is hydrogen, methyl or the radical $COR_5$;
$R_2$ is hydrogen or a radical $COR_5$ or $CO\text{-}Z\text{-}R_6$;
$R_3$ is hydrogen, halogen, trifluoromethyl, trichloromethyl, COOH, $COOCH_3$, OH or nitro;
$R_4$ is hydrogen, halogen, methoxy or methyl;
$R_5$ is $C_1$-$C_6$alkyl, unsubstituted or substituted by one or more halogen atoms, $C_1$-$C_6$alkyl which is interrupted by oxygen or sulphur, $C_1$-$C_6$alkyl which is substituted by one or more halogen atoms and interrupted by oxygen or sulphur, $C_2$-$C_4$alkenyl, unsubstituted or substituted by one or more halogen atoms, phenyl, benzyl, or phenyl or benzyl each substituted by halogen, methyl, trifluoromethyl or trichloromethyl, a 5- or 6-membered heterocycle which contains nitrogen, oxygen or sulphur as hetero atoms, a 3- to 6-membered cycloalkyl radical, or a cycloalkyl radical which is substituted by one or more halogen atoms or methyl groups;
$R_6$ is $C_1$-$C_5$alkyl, phenyl or, if Z is the CO group, is O-alkyl containing 1 or 2 carbon atoms; and
Z is oxygen, sulphur or the CO group;

with the exception of 1-(pyridin-2'-yl)-2-(2,6-dichloroisonicotinoyl)hydrazine, which comprises

a) reacting a 2-hydrazinopyridine derivative of formula II

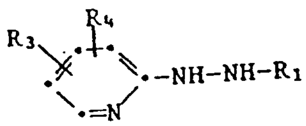

(II)

with a 2,6-dihaloisonicotinoyl derivative of formula III

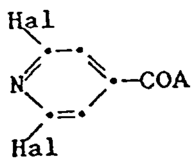

(III)

in an inert solvent, or

b) reacting a 2,6-dihaloisonicotinoyl hydrazide derivative of formula IV

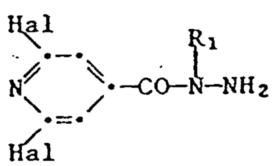

(IV)

with a substituted 2-halopyridine derivative of formula V

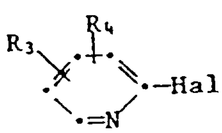

(V)

in an inert solvent and with or without a catalyst, and subsequently

c) reacting the resultant compound of formula Ia

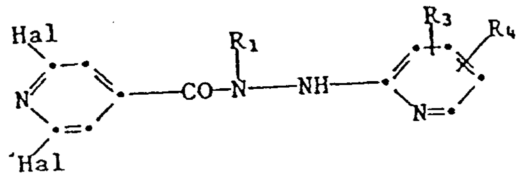

(Ia)

with a compound of formula VI

$R_2CO$-Y (VI)

in an inert solvent and in the absence or presence of a base, in which formulae A is halogen, O-alkyl having from 1 to 4 carbon atoms or S-alkyl having from 1 to 4 carbon atoms, Y is halogen or O-alkyl having from 1 to 4 carbon atoms and Hal and $R_1$ to $R_4$ are as defined for formula I.

**2.** A process according to claim 1 for the preparation of a compound of formula I, wherein

| | |
|---|---|
| Hal | is chlorine or bromine; |
| $R_1$ | is hydrogen; |
| $R_2$ | is hydrogen or a group $COCH_3$, $COC_2H_5$ or 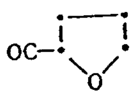 ; and |
| $R_3$ and $R_4$ | are each independently of the other hydrogen, halogen or trifluoromethyl. |

**3.** A process according to claim 1 for the preparation of a compound of formula I, wherein

| | |
|---|---|
| Hal | is chlorine; |
| $R_1$ | is hydrogen; |
| $R_2$ | is hydrogen; and |
| $R_3$ and $R_4$ | are each independently of the other hydrogen, halogen or trifluoromethyl. |

**4.** A process according to claim 1 for the preparation of a compound of formula I, wherein

| | |
|---|---|
| Hal | is chlorine; |
| $R_1$ | is hydrogen; |
| $R_2$ | is a group $COCH_3$, $COC_2H_5$ or 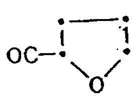 ; and |
| $R_3$ and $R_4$ | are each independently of the other hydrogen, halogen or trifluoromethyl. |

**5.** A process according to claim 1 for the preparation of a compound of formula I, wherein

| | |
|---|---|
| Hal | is chlorine; |
| $R_1$ | is hydrogen; |
| $R_2$ | is hydrogen; |
| $R_3$ | is hydrogen or 3-chloro; and |
| $R_4$ | is hydrogen or 5-trifluoromethyl. |

6. A process according to claim 1 for the preparation of a compound of formula I, wherein
Hal is chlorine;
$R_1$ is hydrogen;
$R_2$ is a group $COCH_3$, $COC_2H_5$ or

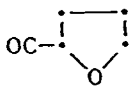 ;

$R_3$ is hydrogen or 3-chloro; and
$R_4$ is hydrogen or 5-trifluoromethyl.

7. A process according to claim 1 for the preparation of a compound of formula I selected from the group
1-acetyl-1-pyridin-2'-yl)-2-(2,6-dichloroisonicotinoyl)hydrazine;
1-propionyl-1-pyridin-2'-yl)-2-(2,6-dichloroisonicotinoyl)hydrazine;
1-tetrahydrofuroyl-1-pyridin-2'-yl)-2-(2,6-dichloroisonicotinoyl)hydrazine;
1-(3'chloro-5'-trifluoromethylpyridin-2'-yl)-(2,6-dichloroisonicotinoyl)hydrazine;
1-methyl-1-(3'-chloro-5'-trifluoromethylpyridin-2'-yl)-(2,6-dichloroisonicotinoyl)hydrazine;
1-ethyl-1-(3'-chloro-5'-trifluoromethylpyridin-2'-yl)-(2,6-dichloroisonicotinoyl)hydrazine.

8. A composition for protecting plants against attack by microorganisms, which comprises as active component at least one compound of formula I as claimed in claim 1, including 1-(pyridin-2'-yl)-2-(2,6-dichloroisonicotinoyl)hydrazine, together with conventional carriers and adjuvants.

9. A composition according to claim 8, which comprises as active component at least one compound of formula I as claimed in any one of claims 2 to 6.

10. A composition according to claim 8, which comprises as active component at least one compound of formula I as claimed in claim 7.

11. A process for the preparation of an agrochemical composition as claimed in claim 8, which comprises intimately mixing at least one compound of formula I as defined in claim 1, including 1-(pyridin-2'-yl)-2-(2,6-dichloroisonicotinoyl)hydrazine, with suitable solid or liquid carriers and adjuvants.

12. A method of protecting plants against attack by phytopathogenic microorganisms, which comprises applying as active ingredient to said plants or to the locus thereof a compound of formula I, including 1-(pyridin-2'-yl)-2-(2,6-dichloroisonicotinoyl)hydrazine, as claimed in claim 1.

13. A method of protecting plants against attack by phytopathogenic microorganisms, which comprises applying as active ingredient to said plants or to the locus thereof a compound of formula I as claimed in any one of claims 2 to 7.

14. A method of immunising plants against attack by phytopathogenic microorganisms, which comprises applying as active ingredient to said plants or to the locus thereof a compound of formula I, including 1-(pyridin-2'-yl)-2-(2,6-dichloroisonicotinoyl)hydrazine, as claimed in claim 1.

15. A method of immunising plants against attack by phytopathogenic microorganisms, which comprises applying as active ingredient to said plants or to the locus thereof a compound of formula I as claimed in any one of claims 2 to 7.

16. A method according to either claim 12 or claim 14, wherein the phytopathogenic microorganisms are fungi.

17. A method according to claim 16, wherein the fungi are from the classes Ascomycetes, Basidiomycetes or Fungi imperfecti.

**18.** A method according to either claim 12 or claim 14, wherein the phytopathogenic microorganisms are bacteria.

**19.** A method according to either claim 12 or claim 14, wherein the phytopathogenic microorganisms are viruses.

**20.** The use of a compound of formula I, including 1-(pyridin-2'-yl)-2-(2,6-dichloroisonicotinoyl)hydrazine, as claimed in claim 1 for the protection of plants against attack by phytopathogenic microorganisms.

**21.** The use of a compound of formula I as claimed in any one of claims 2 to 7 for the protection of plants against attack by phytopathogenic microorganisms.

**Revendications**

**Revendications pour les Etats contractants suivants : AT, BE, CH, DE, FR, GB, GR, IT, LI, LU, NL**

**1.** Composés de formule I

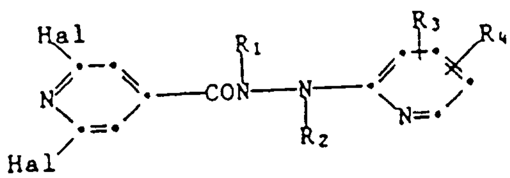

(I)

dans laquelle

Hal représente un halogène ;

$R_1$ représente un hydrogène, méthyle, ou le radical $COR_5$ ;

$R_2$ représente un hydrogène ou l'un des radicaux $COR_5$ ou $CO\text{-}Z\text{-}R_6$ ;

$R_3$ représente un hydrogène, un halogène, un trifluorométhyle, un trichlorométhyle, COOH, $COOCH_3$, OH ou un nitro ;

$R_4$ représente un hydrogène, halogène, méthoxy ou méthyle ;

$R_5$ représente un alcoyle en $C_{1-6}$, alcoyle en $C_{1-6}$ mono- ou poly-substitué par un halogène, un alcoyle en $C_{1-6}$ interrompu par un oxygène ou un soufre, un alcoyle en $C_{1-6}$ mono- ou polysubstitué par un halogène et interrompu par un oxygène ou un soufre, un alcényle en $C_{2-4}$, un alcényle en $C_{2-4}$ mono- ou polysubstitué par un halogène, un phényle, un phényle substitué par un halogène, un méthyle, un trifluorométhyle ou un trichlorométhyle, un benzyle, un benzyle substitué par un halogène, un méthyle, un trifluorométhyle ou un trichlorométhyle, un hétérocycle à 5 ou 6 chaînons avec de l'azote, de l'oxygène ou du soufre comme hétéroatomes, un cycloalcoyle à 3 à 6 chaînons ou un cycloalcoyle mono- ou polysubstitué par un halogène ou un méthyle ;

$R_6$ représente un alcoyle en $C_{1-5}$, un phényle, ou si Z représente le groupe CO, un O-alcoyle en $C_{1-2}$ ; et

Z représente un oxygène, un soufre ou le groupe CO ; à l'exception de la 1-(pyridin-2'-yl)-2-(2,6-dichloroisonicotinoyl)-hydrazine.

**2.** Composés de formule I, selon la revendication 1, dans lesquels

Hal représente un chlore ou un brome ;

$R_1$ représente un hydrogène ;

$R_2$ représente un hydrogène ou un des groupes $COCH_3$, $COC_2H_5$ ou

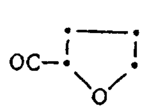

et

$R_3$ et $R_4$ représentent indépendamment l'un de l'autre un hydrogène, halogène ou trifluorométhyle

thyle.

**3.** Composés de formule I selon la revendication 1, dans lesquels
Hal représente un chlore ;
$R_1$ représente un hydrogène ;
$R_2$ représente un hydrogène ; et
$R_3$ et $R_4$ représentent indépendamment l'un de l'autre un hydrogène, un halogène ou un trifluorométhyle.

**4.** Composés de formule I selon la revendication 1, dans lesquels :
Hal représente un chlore ;
$R_1$ représente un hydrogène ;
$R_2$ représente l'un des groupes $COCH_3$, $COC_2H_5$ ou

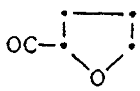

et
$R_3$ et $R_4$ représentent indépendamment l'un de l'autre un hydrogène, halogène ou trifluorométhyle.

**5.** Composés de formule I selon la revendication 1, dans lesquels :
Hal représente un chlore ;
$R_1$ représente un hydrogène ;
$R_2$ représente un hydrogène ;
$R_3$ représente un hydrogène ou un 3-chloro ; et
$R_4$ représente un hydrogène ou un 5-trifluorométhyle.

**6.** Composés de formule I selon la revendication 1, dans lesquels :
Hal est un chlore ;
$R_1$ est un hydrogène ;
$R_2$ représente l'un des groupes $COCH_3$, $COC_2H_5$ ou

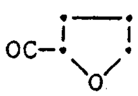

$R_3$ représente un hydrogène ou un 3-chloro ; et
$R_4$ représente un hydrogène ou un 5-trifluorométhyle.

**7.** Composés de formule I selon la revendication 1, choisis dans le groupe :
1-acétyl-1-pyridin-2'-yl)-2-(2,6-dichloroisonicotinoyl)-hydrazine ;
1-propionyl-1-pyridin-2'-yl)-2-(2,6-dichloroisonicotinoyl)-hydrazine ;
1-tétrahydrofuroyl-1-pyridin-2'-yl)-2-(2,6-dichloroisonicotinoyl)-hydrazine ;
1-(3'-chloro-5'-trifluorométhylpyridin-2'-yl)-(2,6-dichloroisonicotinoyl)-hydrazine ;
1-méthyl-1-(3'-chloro-5'-trifluorométhylpyridin-2'-yl)-(2,6-dichloroisonicotinoyl)-hydrazine ;
1-éthyl-1-(3'-chloro-5'-trifluorométhylpyridin-2'-yl)-(2,6-dichloroisonicotinoyl)-hydrazine.

**8.** Procédé de préparation des composés de formule I selon la revendication 1, caractérisé en ce que :

a) on fait réagir des dérivés de 2-hydrazino-pyridine de formule II

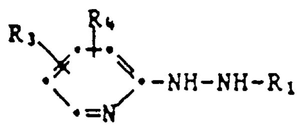 (II)

avec des dérivés de 2,6-dihaloinsonicotinoyle de formule III

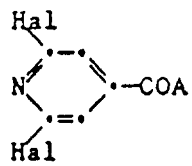 (III)

dans un solvant inerte, ou bien
b) On fait réagir des dérivés d'hydrazide 2,6-dihaloisonicotinique de formule IV

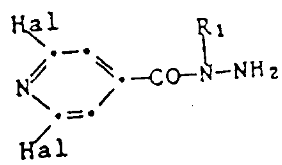 (IV)

avec des dérivés de 2-halopyridine substitués de formule V

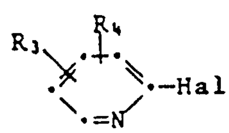 (V)

dans un solvant inerte, éventuellement en présence d'un catalyseur, puis
c) On fait réagir les composés obtenus de formule Ia

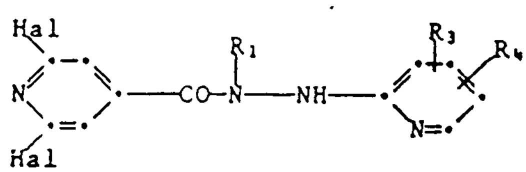 (Ia)

avec des composés de formule VI

$R_2CO$-Y (VI)

dans un solvant inerte, éventuellement en présence d'une base, A représentant un halogène, un 0-alcoyle en $C_{1-4}$ ou un S-alcoyle en $C_{1-4}$, et Y représentant un halogène ou un O-alcoyle en $C_{1-4}$, et Hal et $R_1$ à $R_4$ ayant les significations données sous la formule I.

**9.** Agent de protection des plantes contre l'attaque par les microorganismes, caractérisé en ce qu'il contient outre les supports et additifs habituels, comme composant actif, au moins un composé de formule I selon la revendication 1, y compris la 1-(pyridin-2'-yl)-2-(2,6-dichloroisonicotinoyl)-hydrazine.

**10.** Agent selon la revendication 9, caractérisé en ce qu'il contient comme composant actif au moins un composé de formule I selon les revendications 2-6.

**11.** Agent selon la revendication 9, caractérisé en ce qu'il contient comme composant actif au moins un composé de formule I selon la revendication 7.

**12.** Procédé de préparation d'un agent agrochimique tel que revendiqué dans la revendication 9, caractérisé en ce qu'on mélange au moins un composé de formule I défini selon la revendication 1, y compris la 1-(pyridin-2'-yl)-2-(2,6-dichloroisonicotinoyl)-hydrazine avec des supports et additifs solides ou liquides appropriés.

**13.** Application de composés de formule I, y compris de 1-(pyridin-2'-yl)-2-(2,6-dichloroisonicotinoyl)-hydrazine selon la revendication 1, à la protection des plantes contre l'attaque par les microorganismes phytopathogènes.

**14.** Application des composés de formule I, selon l'une des revendications 2-7 à la protection des plantes contre l'attaque par les microorganismes phytopathogènes.

**15.** Procédé de protection des plantes contre l'attaque par les microorganismes phytopathogènes, caractérisé en ce qu'on applique comme matière active un composé de formule I y compris la 1-(pyridin-2'-yl)-2-(2,6-dichloroisonicotinoyl)-hydrazine selon la revendication 1 sur les plantes ou l'endroit où elles se trouvent.

**16.** Procédé de protection des plantes contre l'attaque par les microorganismes phytophatogènes, caractérisé en ce qu'on applique comme applique comme matière active un composé de formule I selon l'une des revendications 2-7 sur les plantes ou l'endroit où elles se trouvent.

**17.** Procédé d'immunisation des plantes contre l'attaque par les microorganismes phytopathogènes, caractérisé en ce qu'on applique comme matière active un composé de formule I y compris la 1-(pyridin-2'-yl)-2-(2,6-dichloroisonicotinoyl)-hydrazine selon la revendication 1 sur les plantes ou l'endroit où elles se trouvent.

**18.** Procédé d'immunisation des plantes contre l'attaque par les microorganismes phytopathogènes, caractérisé en ce qu'on applique comme matière active un composé de formule I selon l'une des revendications 2-7 sur les plantes ou l'endroit où elles se trouvent.

**19.** Procédé selon les revendications 15 et 17, caractérisé en ce qu'il s'agit, pour les microorganismes phytopathogènes, d'organismes de type champignon.

**20.** Procédé selon la revendication 19, caractérisé en ce que les organismes de type champignon appartiennent aux classes des Ascomycètes, Basidiomycètes, ou Fungi imperfecti.

**21.** Procédé selon les revendications 15 et 17, caractérisé en ce que, pour les microorganismes phytopathogènes, il s'agit de bactéries.

**22.** Procédé selon les revendications 15 et 17, caractérisé en ce qu'il s'agit pour les microorganismes phytopathogènes, de virus.

**Revendications pour l'Etat contractant suivant : ES**

**1.** Procédé de préparation de composés de formule I

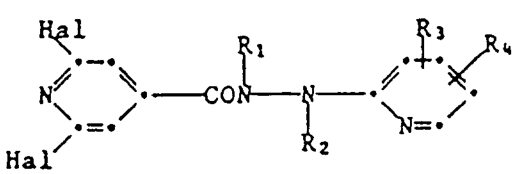

(I)

dans laquelle

Hal représente un halogène ;

$R_1$ représente un hydrogène, méthyle, ou le radical $COR_5$ ;

$R_2$ représente un hydrogène ou l'un des radicaux $COR_5$ ou $CO-Z-R_6$ ;

$R_3$ représente un hydrogène, un halogène, un trifluorométhyle, un trichlorométhyle, COOH, $COOCH_3$, OH ou un nitro ;

$R_4$ représente un hydrogène, halogène, méthoxy ou méthyle ;

$R_5$ représente un alcoyle en $C_{1-6}$, alcoyle en $C_{1-6}$ mono- ou poly-substitué par un halogène, un alcoyle en $C_{1-6}$ interrompu par un oxygène ou un soufre, un alcoyle en $C_{1-6}$ mono- ou polysubstitué par un halogène et interrompu par un oxygène ou un soufre, un alcényle en $C_{2-4}$, un alcényle en $C_{2-4}$ mono- ou polysubstitué par un halogène, un phényle, un phényle substitué par un halogène, un méthyle, un trifluorométhyle ou un trichlorométhyle, un benzyle, un benzyle substitué par un halogène, un méthyle, un trifluorométhyle ou un trichlorométhyle, un hétérocycle à 5 ou 6 chaînons avec de l'azote, de l'oxygène ou du soufre comme hétéroatomes, un cycloalcoyle à 3 à 6 chaînons ou un cycloalcoyle mono- ou polysubstitué par un halogène ou un méthyle ;

$R_6$ représente un alcoyle en $C_{1-5}$, un phényle, ou si Z représente le groupe CO, un O-alcoyle en $C_{1-2}$ ; et

Z représente un oxygène, un soufre ou le groupe CO ; à l'exception de la 1-(pyridin-2'yl)-2-(2,6-dichloroisonicotinoyl)-hydrazine

caractérisé en ce que :

a) ou bien on fait réagir des dérivés de 2-hydrazinopyridine de formule II

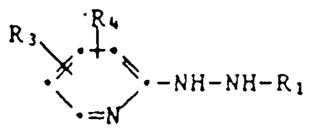

(II)

avec des dérivés de 2,6-dihaloinsonicotinoyle de formule III

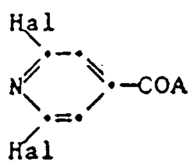

(III)

dans un solvant inerte, ou bien

b) On fait réagir des dérivés d'hydrazide 2,6-dihaloisonicotinique de formule IV

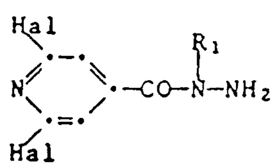

(IV)

avec des dérivés de 2-halopyridine substitués de formule V

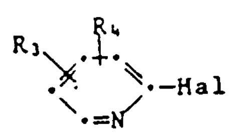

(V)

dans un solvant inerte, éventuellement en présence d'un catalyseur, puis

c) On fait réagir les composés obtenus de formule Ia

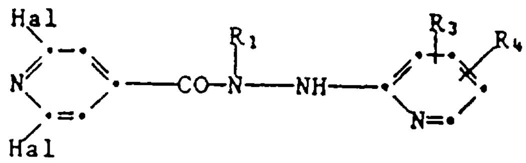 (Ia)

avec des composés de formule VI

$R_2CO\text{-}Y$ (VI)

dans un solvant inerte, éventuellement en présence d'une base, A représentant un halogène, un 0-alcoyle en $C_{1-4}$ ou un S-alcoyle en $C_{1-4}$, et Y représentant un halogène ou un O-alcoyle en $C_{1-4}$, et Hal et $R_1$ à $R_4$ ayant les significations données dans l'introduction sous la formule I.

**2.** Procédé de préparation selon la revendication 1 de composés de formule I, dans laquelle :

Hal représente un chlore ou un brome ;
$R_1$ représente un hydrogène ;
$R_2$ représente un hydrogène ou un des groupes $COCH_3$, $COC_2H_5$ ou

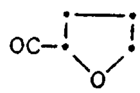

et
$R_3$ et $R_4$ représentent indépendamment l'un de l'autre un hydrogène, halogène ou trifluorométhyle.

**3.** Procédé selon la revendication 1 de préparation des composés de formule I selon la revendication 1, dans lesquels

Hal représente un chlore ;
$R_1$ représente un hydrogène ;
$R_2$ représente un hydrogène ; et
$R_3$ et $R_4$ représentent indépendamment l'un de l'autre un hydrogène, un halogène ou un trifluorométhyle.

**4.** Procédé selon la revendication 1 de préparation des composés de formule I selon la revendication 1, dans lesquels :

Hal représente un chlore ;
$R_1$ représente un hydrogène ;
$R_2$ représente l'un des groupes $COCH_3$, $COC_2H_5$ ou

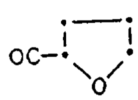

et
$R_3$ et $R_4$ représentent indépendamment l'un de l'autre un hydrogène, halogène ou trifluorométhyle.

**5.** Procédé selon la revendication 1 de préparation des composés de formule I selon la revendication 1, dans lesquels :

Hal représente un chlore ;
$R_1$ représente un hydrogène ;

$R_2$ représente un hydrogène ;
$R_3$ représente un hydrogène ou un 3-chloro ; et
$R_4$ représente un hydrogène ou un 5-trifluorométhyle.

6. Procédé selon la revendication 1 de préparation des composés de formule I selon la revendication 1, dans lesquels :
Hal est un chlore ;
$R_1$ est un hydrogène ;
$R_2$ représente l'un des groupes $COCH_3$, $COC_2H_5$ ou

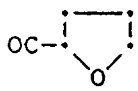

$R_3$ représente un hydrogène ou un 3-chloro ; et
$R_4$ représente un hydrogène ou un 5-trifluorométhyle.

7. Procédé selon la revendication 1 de préparation d'un composé de formule I choisi dans le groupe :
1-acétyl-1-pyridin-2'-yl)-2-(2,6-dichloroisonicotinoyl)-hydrazine ;
1-propionyl-1-pyridin-2'-yl)-2-(2,6-dichloroisonicotinoyl)-hydrazine ;
1-tétrahydrofuroyl-1-pyridin-2'-yl)-2-(2,6-dichloroisonicotinoyl)-hydrazine ;
1-(3'-chloro-5'-trifluorométhylpyridin-2'-yl)-(2,6-dichloroisonicotinoyl)-hydrazine ;
1-méthyl-1-(3'-chloro-5'-trifluorométhylpyridin-2'-yl)-(2,6-dichloroisonicotinoyl)-hydrazine ;
1-éthyl-1-(3'-chloro-5'-trifluorométhylpyridin-2'-yl)-(2,6-dichloroisonicotinoyl)-hydrazine.

8. Agent de protection des plantes contre les microorganismes, caractérisé en ce qu'il contient, outre les supports et additifs habituels, comme composants actifs, au moins un composé de formule I selon la revendication 1, y compris la 1-(pyridin-2'-yl)-2-(2,6-dichloroisonicotinoyl)-hydrazine.

9. Agent selon la revendication 8, caractérisé en ce qu'il contient comme composant actif au moins un composé de formule I selon les revendications 2-6.

10. Agent selon la revendication 8, caractérisé en ce qu'il contient comme composant actif au moins un composé de formule I selon la revendication 7.

11. Procédé de préparation d'un agent agrochimique tel que revendiqué dans la revendication 8, caractérisé en ce qu'on mélange intimement au moins un composé de formule I défini selon la revendication 1, y compris la 1-(pyridin-2'-yl)-2-(2,6-dichloroisonicotinoyl)-hydrazine avec des supports et additifs solides ou liquides appropriés.

12. Procédé de protection des plantes contre l'attaque par les microorganismes phytopathogènes, caractérisé en ce qu'on applique comme matière active un composé de formule I y compris la 1-(pyridin-2'-yl)-2-(2,6-dichloroisonicotinoyl)hydrazine selon la revendication 1 sur les plantes ou l'endroit où elles se trouvent.

13. Procédé de protection des plantes contre l'attaque par des microorganismes phytopathogènes, caractérisé en ce qu'on applique comme matière active un composé de formule I selon l'une des revendications 2-7 sur les plantes ou l'endroit où elles se trouvent.

14. Procédé d'immunisation des plantes contre l'attaque par les microorganismes phytopathogènes, caractérisé en ce qu'on applique comme matière active un composé de formule I y compris la 1-(pyridin-2'-yl)-2-(2,6-dichloroisonicotinoyl)-hydrazine selon la revendication 1 sur les plantes ou l'endroit où elles se trouvent.

15. Procédé d'immunisation des plantes contre l'attaque par les microorganismes phytopathogènes, caractérisé en ce qu'on applique comme matière active un composé de formule I selon l'une des revendications 2 à 7 sur les plantes ou l'endroit où elles se trouvent.

**16.** Procédé selon les revendications 12 et 14, caractérisé en ce qu'il s'agit, pour les microorganismes phytopathogènes, d'organismes de type champignon.

**17.** Procédé selon la revendication 16, caractérisé en ce qu'il s'agit d'organismes du type champignon des classes Ascomycètes, Basidiomycètes, ou Fungi imperfecti.

**18.** Procédé selon les revendications 12 et 14, caractérisé en ce qu'il s'agit, pour les microorganismes phytopathogènes, de bactéries.

**19.** Procédé selon les revendications 12 et 14, caractérisé en ce qu'il s'agit, pour les microorganismes phytopathogènes, de virus.

**20.** Application de composés de formule I, y compris de 1-(pyridin-2'-yl)-2-(2,6-dichloroisonicotinoyl)-hydrazine à la protection des plantes contre l'attaque par les microorganismes phytopathogènes.

**21.** Application des composés de formule I selon l'une des revendications 2 à 7 à la protection des plantes contre l'attaque par les microorganismes phytopathogènes.